# EUROPÄISCHE PATENTSCHRIFT

(11) **EP 1 680 515 B1**
(45) Veröffentlichungstag und Bekanntmachung des Hinweises auf die Patenterteilung: **18.11.2009**
(21) Anmeldenummer: 04797232.8
(22) Anmeldetag: 05.11.2004
(51) Int. Cl.: C12Q 1/68, G01N 11/16

(54) **CHEMISCHE ANALYSE MIT DYNAMISCHER VISKOSIMETRIE**
CHEMICAL ANALYSIS USING DYNAMIC VISCOMETRY
ANALYSE CHIMIQUE AVEC VISCOSIMETRIE DYNAMIQUE

(30) Priorität: 06.11.2003 CH 192303
(43) Veröffentlichungstag der Anmeldung: 19.07.2006
(73) Patentinhaber: Dual, Jürg, 8126 Zumikon (CH); Bestmann, Lukas, 8406 Winterthur (CH); Häusler, Klaus, 8053 Zürich (CH)
(72) Erfinder: Dual, Jürg, 8126 Zumikon (CH); Bestmann, Lukas, 8406 Winterthur (CH); Häusler, Klaus, 8053 Zürich (CH)
(74) Vertreter: Wiedmer, Edwin
(86) Internationale Anmeldenummer: PCT/CH2004/000675
(87) Internationale Veröffentlichungsnummer: WO 2005/045068

(56) Entgegenhaltungen:
- DE-A1- 3 615 553
- US-A- 4 920 787
- US-A- 5 837 885
- GOODMAN A ET AL: "Effect of Length, Topology, and Concentration on the Microviscosity and Microheterogeneity of DNA Solutions" JOURNAL OF MOLECULAR BIOLOGY, LONDON, GB, Bd. 323, Nr. 2, 18. Oktober 2002 (2002-10-18), Seiten 199-215, XP004449864 ISSN: 0022-2836
- DATABASE WPI Week 1987 Derwent Publications Ltd., London, GB; AN 1987-270360 XP002315289 & SU 1 289 870 A (SAMOTSVEROV A R) 15. Februar 1987 (1987-02-15)
- SUH D ET AL: "Criteria for the mode of binding of DNA binding agents." BIOORGANIC & MEDICINAL CHEMISTRY. JUN 1995, Bd. 3, Nr. 6, Juni 1995 (1995-06), Seiten 723-728, XP002315287 ISSN: 0968-0896
- PARODI S ET AL: "A circular channel crucible oscillating viscometer. Detection of DNA damage induced in vivo by exceedingly small doses of dimethylnitrosamine" JOURNAL OF MOLECULAR BIOLOGY 1981 UNITED KINGDOM, Bd. 147, Nr. 4, 1981, Seiten 501-521, XP009042966
- BERNENGO J C ET AL: "AN AUTOMATIC RECORDING DEVICE FOR ZIMM CROTHERS VISCOMETER" REVIEW OF SCIENTIFIC INSTRUMENTS, Bd. 44, Nr. 3, 1973, Seiten 311-313, XP002315288 ISSN: 0034-6748

## Beschreibung

### Technisches Gebiet

Die vorliegende Erfindung betrifft ein Verfahren und eine Vorrichtung zum Ermitteln des Reaktionszustands einer chemischen Reaktion, insbesondere einer Reaktion zur Amplifikation von Nukleinsäuren.

### Stand der Technik/Hintergrund der Erfindung

Bei chemischen Reaktionen ist es meist erwünscht, das Reaktionsergebnis durch Messung geeigneter Grössen zu quantifizieren oder den Reaktionsverlauf anhand einer solchen Grösse zu verfolgen. Ein bekanntes Beispiel ist das Verfolgen von Farbänderungen eines Farbindikators, der einem Reaktionsgemisch beigefügt wurde.

Bei einem Amplifikationsprozess von Nukleinsäuren wird im Gegensatz zu üblichen chemischen Reaktion ein Edukt nicht in ein komplett anderes Produkt umgewandelt, sondern ein Teil eines Moleküls mit Hilfe anderer Edukte vervielfältigt. Speziell bei Verfahren zur Amplifikation von Nukleinsäuren, insbesondere der Polymerase-Kettenreaktion (engl. Polymerase Chain Reaction, PCR) wird das Reaktionsergebnis heute häufig durch Anfärben mit Ethidiumbromid und Detektion des Fluoreszenzsignals quantifiziert. Es wurde auch vorgeschlagen, den Hybridisierungsvorgang selbst durch Fluoreszenz zu detektieren. Ein solches Verfahren ist in der US 6,174,670 offenbart. Das detektierte Fluoreszenzsignal resultiert aus einem Fluoreszenz-Resonanz-Energietransfer (FRET) zwischen zwei Fluorophoren bei Excitation des Donorfluorophoren mit Licht. Durch die Analyse von spezifischen Schmelz- und Erstarrungskurven kann die DNA genotypisiert werden.

Eine Überwachung des Reaktionsveriaufs durch Fluoreszenz ist relativ aufwändig, da ein empfindliches optisches System zur Detektion bereitgestellt werden muss. Zudem ist ein solches Verfahren wegen der dazu notwendigen Fluoreszenzmarker im Gebrauch kostenintensiv.

Es ist auch bekannt, den Verlauf von viskositätsändemden Reaktionen, z.B. Polymerisationsreaktionen, mittels Viskosimetrie zu untersuchen. Eine Reaktion ist viskositätsändemd, falls die Viskosität des Produktes oder der Mischung der Produkte von der Viskosität der Mischung der Edukte abweicht. Dann wird sich die Viskosität des Gemisches mit zunehmendem Reaktionsfortschritt der Viskosität der Produkte annähern. Die Viskositätsmessung liefert dann ein Mass für den Reaktionsfortschritt. Die hierfür eingesetzten Viskosimeter sind jedoch in der Regel für grosstechnische Produktionsanlagen optimiert und ungeeignet, um kleine Reaktionsvolumina zu untersuchen.

A. Goodman et al, "Effect of Length, Topology, and Concentration on the Microviscosity and Microheterogeneity of DNA Solutions", Journal of Molecular Biology, London, GB, Bd. 323, Nr. 2, 18. Oktober 2002, Seiten 199-215, XP004449864, ISSN 0022-2836 beschreibt die Bestimmung einer mikroskopischen Viskosität von DNA-Lösungen mittels Mehrteilchen-Nanotracking. Hierzu werden fluoreszente Mikrokügelchen mikroskopisch überwacht. Das Verfahren ist sehr kompliziert und erfordert eine Kamera, die auf einem speziellen Mikroskop montiert ist. Es eignet sich daher nicht für Routineuntersuchungen.

### Darstellung der Erfindung

Es ist daher eine Aufgabe der vorliegenden Erfindung, ein Verfahren zum Ermitteln des Reaktionszustands eines chemischen Reaktionsvorgangs in einem Reaktionsgemisch anzugeben, wobei der chemische Reaktionsvorgang eine Amplifikationsreaktion für Nukleinsäuren umfasst. Das Verfahren soll einfach durchführbar und kostengünstig sein. Diese Aufgabe wird gelöst durch ein Verfahren mit den Merkmalen des Anspruchs 1.

Das erfindungsgemässe Verfahren umfasst eine Viskositätsbestimmung des Reaktionsgemisches. Diese kann mit einem geeigneten Viskosimeter, bevorzugt mit einem dynamischen Viskosimeter erfolgen. Es kann aber auch ein andersartiges Viskosimeter Verwendung finden, z.B. ein Kapillarviskosimeter, wie es im Stand der Technik bekannt ist.

Vorteilhafte Ausgestaltungen der Erfindung sind in den abhängigen Ansprüchen angegeben.

Im erfindungsgemässen Verfahren wird der Reaktionszustand, insbesondere das Ergebnis, einer chemischen Reaktion ermittelt. Die chemische Reaktion umfasst mindestens einen Reaktionsschritt, in dem eine Amplifikation von Nukleinsäuren stattfindet. Das Verfahren zeichnet sich dadurch aus, dass eine Messung der Viskosität vorgenommen wird.

Das erfindungsgemässe Verfahren ermöglicht es, sehr kostengünstig festzustellen, ob und in welchem Ausmass eine Amplifikation tatsächlich stattgefunden hat. Dem liegt die Erkenntnis zugrunde, dass praktisch jede Amplifikationsreaktion zu einer Viskositätsänderung führt, so z.B. eine Polymerase-Kettenreaktion (PCR), eine Ligase-Kettenreaktion (LCR) oder eine NASBA-Reaktion (engl. Nucleic Acid Sequence Based Amplification). Die entsprechenden Verfahren sind in Fachkreisen wohlbekannt.

Das erfindungsgemässe Verfahren ermöglicht insbesondere die sehr kostengünstige Durchführung von Screeningverfahren auf das Vorliegen von Nukleinsäuren bestimmter Organismen oder auf das Vorliegen einer Mutation im Erbgut eines Organismus, insbesondere eines mikrobiellen, pflanzlichen oder tierischen (einschliesslich menschlichen) Organismus. Für die letztgenannte Anwendung umfasst das Verfahren bevorzugt weitere Auswertungsschritte, die es erlauben, das Vorliegen einer Mutation in einer Nukleinsäure zu bestimmen.

In einer bevorzugten Ausführungsform umfasst in diesem Falle die Amplifikationsreaktion eine allelspezifische Amplifikationsreaktion, wie sie in Fachkreisen bekannt ist. Eine allelspezifische Amplifikation ist eine Amplifikation, bei der die Menge der durch die Reaktion erzeugten Nukleinsäurefragmente von der Ab- oder Anwesenheit der Mutation auf der anfänglich vorliegenden Zielsequenz abhängt. Die weiteren Auswertungsschritte umfassen dann bevorzugt einen Schritt, in dem das ermittelte Mass für die Viskosität mit einem zuvor gemessenen oder errechneten Vergleichsmass verglichen wird, wobei das Ergebnis dieses Vergleichs eine Entscheidung über das Vorliegen der Mutation ermöglicht.

Bevorzugt umfasst der Reaktionsvorgang mindestens einen Reaktionsschritt, bei dem Nukleinsäurefragmente miteinander verknüpft werden, bevorzugt in einer Form, bei der die Fragmente miteinander polymerisieren. In einer bevorzugten Ausführungsform erfolgt die Verknüpfung von Ziel-Nukleinsäurefragmenten mittels Linker-Oligonukleotiden. Das Linker-Oligonukleotid ist dazu ausgebildet, mit einem ersten Sondenbereich nahe seinem 5'-Ende auf einem ersten Nukleinsäurefragment zu hybridisieren und mit einem zweiten Sondenbereich nahe seinem 3'-Ende auf einem zweiten Nukleinsäurefragment zu hybridisieren. Bevorzugt werden die Linker-Oligonukleotide selbst anschliessend an einen solchen Hybridisationsvorgang mittels einer Ligase verknüpft. Dies kann in Form einer LCR erfolgen. Vor der Verknüpfung mit der Ligase kann optional noch eine Elongation mit einer Polymerase ohne 3'→5'-Exonuklease-Aktivität stattfinden, wie sie üblicherweise bei einer sogenannten Gap-LCR Verwendung findet. Das Linker-Oligonukleotid umfasst bevorzugt einen Linker-Bereich, der eine repetitive Anordnung von Basen umfasst, z.B. einen Bereich von bevorzugt 5 bis 20 gleichen Basen, z.B. T-Basen. Beidseitig an diesen Bereich schliessen sich die zwei Sondenbereiche an, deren Nukleotidsequenzen bevorzugt im wesentlichen komplementär zu jeweils einer Zielsequenz sind. Wenn die Verknüpfung der Linker-Oligonukleotide allelspezifisch auf einen Single Nucleotide Polymorphism (SNP, Austausch einer einzigen Base an einer Mutationsstelle) erfolgen soll, ist bevorzugt das Linker-Oligonukleotid so gewählt, dass eine Base, die bevorzugt das 3'-Ende des Linker-Oligonukleotids bildet oder wenige, bevorzugt ein oder zwei, Positionen vom 3'-Ende entfernt ist, zur Base der Zielsequenz an der Mutationsstelle komplementär ist.

Der Linker-Bereich und die daran anschliessenden Bereiche sind so ausgebildet, dass im wesentlichen keine Hybridisierung beider Sondenbereiche an ein einziges Nukleinsäurefragment mit der Zielsequenz erfolgt, sondern dass bevorzugt jeder der Sondenbereiche an jeweils ein anderes Nukleinsäurefragment hybridisiert, das die Zielsequenz aufweist. Damit eine Verknüpfung durch die Ligase stattfinden kann, ist bevorzugt das 5'-Ende des Linker-Oligonukleotids phosphoryliert.

Es findet hier also einerseits eine Verknüpfung von Ziel-Nukleinsäurefragmenten durch Linker-Oligonukleotide statt, andererseits zusätzlich eine Verknüpfung der Linker-Oligonukleotide. Bei der Verknüpfung kann ein lineares Makromolekül und/oder ein Netzwerk entstehen. Bezüglich der Begriffe "lineares Makromolekül" und "Netzwerk" wird auf das IUPAC Compendium of Chemical Terminology, 2nd Edition (1997), Bezug genommen. Durch eine solche zusätzliche Polymerisation erhöht sich die Viskosität markant, sofern eine ausreichende Eduktmenge, d.h. eine ausreichende Zahl Fragmente der Zielsequenz, vorliegt.

Die Viskositätsbestimmung erfolgt bevorzugt mit einem dynamischen Viskosimeter, kann aber auch z.B. mit einem bekannten Kapillarviskosimeter erfolgen.

Unter einem dynamischen Viskosimeter ist im Zusammenhang mit diesem Dokument eine Vorrichtung zu verstehen, die einen Resonator umfasst, der zu einer mechanischen Eigenschwingung, bevorzugt einer Torsionsschwingung, fähig ist. Der Resonator weist dabei eine Kontaktoberfläche auf, die mit einem Fluid in Kontakt gebracht werden kann. Mittels eines Wandlers, z.B. eines Piezowandlers oder eines elektromagnetischen Wandlers, können der Resonator und damit die Kontaktoberfläche zu Schwingungen angeregt werden. Das Fluid an der Kontaktoberfläche wird die Schwingung dämpfen. Ein Mass für die Viskosität des Fluids lässt sich dann aus der Dämpfung des Resonators ableiten. Eine quantitative Bestimmung der Viskosität wird möglich, wenn die Dichte des Fluids bekannt ist.

Ein dynamisches Viskosimeter ist einfach im Gebrauch, kostengünstig in der Herstellung und hochempfindlich für kleine Viskositätsänderungen. Während dynamische Viskosimeter an sich bekannt sind, zeichnet sich das erfindungsgemässe Verfahren dadurch aus, dass das dynamische Viskosimeter dazu verwendet wird, durch die Viskosität Aufschluss über den Reaktionszustand zu erhalten. Im Gegensatz zu Verfahren, wie sie typischerweise zur Überwachung grosstechnischer Polymerisationsreaktionen eingesetzt werden, eignet sich das erfindungsgemässe Verfahren besonders für Anwendungen, bei denen die Menge des Reaktionsgemisches verhältnismässig klein ist.

Verschiedene Ausführungsformen dynamischer Viskosimeter sind in der US 4,920,787 und der WO 95/24630 beschrieben. Für die Ausgestaltung eines dynamischen Viskosimeters wird explizit auf die Offenbarung dieser Dokumente Bezug genommen.

Ein grosser Vorteil der Verwendung eines dynamischen Viskosimeters im erfindungsgemässen Verfahren liegt in der hohen Empfindlichkeit, die sich mit dynamischen Viskosimetern erreichen lässt. Selbst kleine Viskositätsänderungen, auf der Skala von wenigen Prozent oder weniger, in kleinen Probenvolumina können mit einem solchen Viskosimeter detektiert werden. Vorzugsweise befindet sich das Reaktionsgemisch in einem Probenraum, der die Kontaktoberfläche des Resonators enthält und dessen Volumen kleiner als 1 Milliliter, bevorzugt kleiner als 100 Mikroliter, in einer vorteilhaften Ausgestaltung kleiner als 10 Mikroliter ist. In der Regel wird das Volumen des Probenraums 1 Mikroliter übersteigen; bei entsprechender Ausführung des Viskosimeters kann das Volumen aber diesen Wert unterschreiten. Um die Messung solch kleiner Probenvolumina mit ausreichender Empfindlichkeit zu ermöglichen, gibt die vorliegende Erfindung auch besondere Ausgestaltungen des Viskosimeters an.

Vorzugsweise ist der Probenraum zumindest teilweise durch Kapillarkräfte begrenzt. Gerade bei kleinen Probenvolumina ist es so sehr einfach, einen genau vorgegebenen Bereich der Kontaktoberfläche mit dem Reaktionsgemisch in Kontakt zu bringen und dort während der Messung zu halten.

Beim erfindungsgemässen Verfahren kann ein Reaktionsvorgang stattfinden, während sich das Reaktionsgemisch im Probenraum des Viskosimeters befindet, oder das Reaktionsgemisch wird erst nach Ablauf der Reaktion in das Viskosimeter eingebracht. Vorteile ergeben sich, wenn wenigstens ein Teil des Reaktionsvorgangs stattfindet, während sich das Reaktionsgemisch in Kontakt mit der Kontaktoberfläche befindet, da dann aufinrändige Probentransfers entfallen können. Dies ist besonders dann von Vorteil, wenn es sich bei dem Reaktionsvorgang um eine Amplifikationsreaktion für Nukleinsäuren handelt, da so eine mögliche Kontamination vermieden wird und ein aufwändiger manueller Schritt in der Handhabung entfallen kann. Auch kann durch periodische Messung der Viskosität direkt das Fortschreiten der Reaktion verfolgt werden. Bevorzugt wird in diesem Fall die Kontaktoberfläche durch die Innenseite eines Glasrohrs gebildet, und auf dem Glasrohr ist vorteilhaft ein Temperierelement, z.B. ein Heizelement oder ein sowohl Heizung als auch Kühlung erlaubendes Peltierelement, aufgedampft.

Im folgenden Text wird auch eine Vorrichtung zur Ermittlung des Reaktionszustands eines viskositätsändernden chemischen Reaktionsvorgangs in einem Reaktionsgemisch beschrieben. Diese zeichnet sich dadurch aus, dass sie ein dynamisches Viskosimeter mit einem Torsionsschwinger als Resonator umfasst. Mit anderen Worten umfasst die Vorrichtung einen Resonator, der eine Kontaktoberfläche aufweist und zu einer Torsionsschwingung parallel zur Kontaktoberfläche fähig ist, sowie mindestens einen ersten mit dem Resonator zusammenwirkenden Wandler.

Häufig ist es erwünscht, die Temperatur des Reaktionsgemisches auf einen definierten Wert einzustellen, sei es, um definierte Messbedingungen zu erreichen, sei es, um Reaktionen bei erhöhten Temperaturen zu induzieren. Hierzu umfasst das Viskosimeter bevorzugt ein nahe der Kontaktoberfläche angeordnetes Temperierelement. Dies kann ein reines Heizelement sein, z.B. ein Heizdraht, bevorzugt ist aber ein Element, das sowohl Heizen als auch Kühlen ermöglicht, z.B. ein Peltierelement. Das Temperierelement ist bevorzugt am Resonator angebracht. Es kann durch Aufdampfen von Metallschichten auf den Resonator hergestellt sein, insbesondere, wenn dieser ein Glasrohr umfasst.

Das Temperierelement kann als stromdurchflossenes Element gleichzeitig dazu dienen, durch die in einem äusseren Magnetfeld wirkende Lorentzkraft eine Kraft bzw. ein Drehmoment auf den Resonator auszuüben. In dieser Funktion kann das Temperierelement Teil des (elektromagnetischen) Wandlers sein. Umgekehrt führt eine Schwingung des Resonators in einem äusseren Magnetfeld dann zu induzierten Spannungen im Temperierelement, die zur Detektion der Schwingung dienen können. In diesem Falle umfasst das Viskosimeter bevorzugt Mittel zur Erzeugung eines statischen Magnetfelds, insbesondere einen oder mehrere Permanentmagneten, in der Umgebung des Temperierelements.

Vorteilhaft umfasst der Resonator ein (Mineral-) Glasrohr mit einem Aussendurchmesser von 1 Millimeter oder weniger. Bevorzugt liegt der Aussendurchmesser bei 0,5 Millimeter oder weniger. Der Innendurchmesser liegt bevorzugt bei 0,8 Millimeter oder weniger, besonders bevorzugt bei 0,4 Millimeter oder weniger. Die Wandstärke liegt bevorzugt zwischen 0,02 Millimeter und 0,1 Millimeter, besonders bevorzugt bei etwa 0,05 Millimeter. Bevorzugt besteht das Glasrohr aus Quarzglas. Ein Resonator mit einem solchen Glasrohr weist eine hohe Empfindlichkeit auf, unter anderem aufgrund der besonderen mechanischen Eigenschaften von Gläsern, insbesondere von Quarzglas, und aufgrund der niedrigen spezifischen Masse im Vergleich zu vielen metallischen Werkstoffen. Ausserdem ist Glas in hohem Masse chemisch inert, wodurch verhindert wird, dass das Resonatormaterial durch das Reaktionsgemisch angegriffen oder chemisch verändert wird. Weiterhin eignet sich ein Glasrohr besonders gut zum Aufdampfen von Metallbereichen, wodurch besonders kostengünstig ein Temperierelement hergestellt werden kann. Dies ist besonders dann interessant, wenn der Resonator als Wegwerfprodukt konzipiert ist.

Das Glasrohr ist bevorzugt an einer ersten und einer zweiten Inertialmasse befestigt, deren Masse jeweils ein Vielfaches (wenigstens das Zehnfache) der Masse des Glasrohrs beträgt. Jede Inertialmasse ist direkt oder indirekt, z.B. über einen abgeschwächten Bereich, der als Federelement wirkt, mit einem Gehäuse oder Halter verbunden. Bezüglich weiterer Überlegungen zur Inertialmasse und deren Verbindung mit dem Gehäuse bzw. Halter wird ausdrücklich auf die Offenbarung der US 4,920,787 und WO 95/24630 Bezug genommen. Der Wandler ist im Bereich zwischen den Inertialmassen nahe einem Schwingungsantiknoten (Schwingungsbauch, Bereich maximaler Amplitude) einer Eigenschwingung des Resonators angeordnet. Bevorzugt befindet sich der Wandler in der Mitte zwischen den Inertialmassen. Das Innere des Rohrs bildet die Kontaktoberfläche für das Reaktionsgemisch.

Das Glasrohr ist vorzugsweise lösbar an den Inertialmassen befestigt, z.B. mittels einer Klemmbuchse. Damit wird der Resonator leicht auswechselbar, was es leicht ermöglicht, diesen nach jeder Messung aus Hygienegründen zu ersetzen. In diesem Falle ist es besonders vorteilhaft, wenn stromdurchflossene Elemente auf dem Glasrohr vorhanden sind, die Teil des Wandlers sind und zur Magnetfelderzeugung dienen.

In einer weiteren vorteilhaften Ausgestaltung weist der Resonator ein Rohr und einen Kontaktkörper auf. Dieser ist mit dem Rohr verbunden und weist einen Durchmesser auf, der grösser ist als der Durchmesser des Rohrs, bevorzugt mindestens das Doppelte des Durchmessers des Glasrohrs. Eine äussere Oberfläche des Kontaktkörpers bildet die Kontaktoberfläche. Der Kontaktkörper zeichnet sich dadurch aus, dass er zu einer direkten elektromagnetischen Wechselwirkung mit einem vom Wandler erzeugten Magnetfeld ausgebildet ist, d.h. er ist Teil des (elektromagnetischen) Wandlers.

In einer besonderen Ausgestaltung umfasst der Kontaktkörper dazu einen Permanentmagneten oder wird von einem solchen gebildet. Das Viskosimeter umfasst dann wenigstens ein Paar von Elektromagneten, die so angeordnet sind, dass sie mit dem Permanentmagneten magnetisch wechselwirken. Alternativ kann auch der Kontaktkörper mindestens einen Elektromagneten aufweisen.

In einer weiteren Ausgestaltung umfasst die Vorrichtung eine Dosiervorrichtung für das Reaktionsgemisch, die an einem Halter oder Gehäuse angebracht sein kann oder vom Gehäuse selbst gebildet wird. Die Dosiervorrichtung ist nahe dem Kontaktkörper angeordnet und weist im Bereich der Kontaktoberfläche eine Öffnung auf. Durch diese Öffnung wird das Reaktionsgemisch in einen vorbestimmten Bereich zwischen der Kontaktoberfläche und der Dosiervorrichtung eingebracht. Die Dosiervorrichtung ist so von der Kontaktoberfläche beabstandet, dass das Reaktionsgemisch durch Kapillarkräfte in diesem vorbestimmten Bereich gehalten wird. Dadurch bleibt das Reaktionsgemisch in einem definierten Probenvolumen begrenzt.

Bevorzugt umfasst der Resonator auch in dieser Ausgestaltung ein Rohr mit einem darauf angebrachten Kontaktkörper grösseren Durchmessers. In diesem Falle ist die Öffnung bevorzugt zentral, d.h. im wesentlichen konzentrisch mit dem Rohr, angeordnet.

Bevorzugt wird das Viskosimeter mit einem Phasenregelkreis betrieben, wie er in der US 4,920,787 oder der WO 95/24630 ausführlich beschrieben ist. Dazu weist die Vorrichtung eine Rückkopplungseinrichtung auf, die einen Oszillator umfasst, der den ersten Wandler treibt. Die Rückkopplungseinrichtung ist dazu vorgesehen, den Resonator im Bereich einer Eigenfrequenz zu stabilisieren. Dazu ist ein Ausgang der Rückkopplungseinrichtung zumindest indirekt mit dem ersten Wandler verbunden, welcher den Resonator zu Schwingungen anregt. Zur Detektion der Schwingungen dient ebenfalls derselbe Wandler, oder es ist ein zweiter Wandler zu diesem Zweck am Resonator vorhanden. Ein Eingang der Rückkopplungsvorrichtung ist zumindest indirekt mit dem ersten Wandler bzw. dem zweiten Wandler verbunden. Ein Betrieb mit einem solchen Regelkreis ermöglicht besonders stabile, reproduzierbare Messungen.

Um einen Betrieb zu ermöglichen, wie er in der WO 95/24630 offenbart ist, ist zwischen dem Ausgang der Rückkopplungsvorrichtung und dem ersten Wandler ein erster Schalter und zwischen dem ersten bzw. zweiten Wandler und dem Eingang der Rückkopplungsvorrichtung ein zweiter Schalter vorhanden. Die Schalter können beliebige geeignete elektronische Mittel umfassen, z.B. Transistoren. Die Messung kann dann in einem getakteten Modus erfolgen, ohne die hohe Frequenzstabilität des Phaseregelkreises zu beeinträchtigen.

Bei einer so ausgestalteten Vorrichtung umfasst der Schritt des Bestimmens des Masses für die Viskosität vorzugsweise die folgenden Teilschritte:
- Anregen des ersten Wandlers mit einem von der Rückkopplungseinrichtung erzeugten Signal für ein erstes vorbestimmtes Zeitintervall, wobei mindestens während dieses ersten Zeitintervalls die Verbindung zwischen dem Eingang der Rückkopplungsvorrichtung und dem ersten bzw. zweiten Wandler unterbrochen wird;
- Zuführen eines vom ersten bzw. zweiten Wandler erzeugten Signals zur Rückkopplungseinrichtung während eines zweiten vorbestimmten Zeitintervalls, wobei mindestens während dieses zweiten Zeitintervalls die Verbindung zwischen dem Ausgang der Rückkopplungsvorrichtung und dem ersten Wandler unterbrochen wird.

Diese Betriebsart hat sich als besonders störungsarm erwiesen, wodurch sie besonders für kleine Probenmengen geeignet ist.

Die genannten speziellen Ausgestaltungen eines dynamischen Viskosimeters eignen sich selbstverständlich auch für andere Zwecke als dem Ermitteln des Reaktionszustands einer chemischen Reaktion und weisen darin ähnliche Vorteile auf.

### Kurze Beschreibung der Zeichnungen

Die Erfindung wird im folgenden anhand der Zeichnungen näher beschrieben, in denen zeigt:
- Fig. 1: eine schematische Darstellung einer ersten Gestaltung eines Viskosimeters in Schnittansicht von oben;
- Fig. 2: eine schematische Darstellung des Viskosimeters der Fig. 1 im Querschnitt durch die Ebene 2-2;
- Fig. 3: eine schematische Darstellung einer zweiten Gestaltung eines Viskosimeters im Seitenschnitt;
- Fig. 4: eine schematische Darstellung des Viskosimeters der Fig. 3 im Querschnitt durch die Ebene 4-4;
- Fig. 5: eine schematische Darstellung einer dritten Gestaltung eines Viskosimeters im Seitenschnitt;
- Fig. 6: eine schematische Darstellung des Viskosimeters der Fig. 3 im Querschnitt durch die Ebene 6-6;
- Fig. 7: eine schematische vergrösserte Teildarstellung einer Variante der dritten Gestaltung im Seitenschnitt;
- Fig. 8: eine schematische Seitenansicht eines Resonatorrohrs;
- Fig. 9: eine schematische Darstellung des Resonatorrohrs der Fig. 8 im Querschnitt durch die Ebene 9-9;
- Fig. 10: eine schematische Darstellung einer Variante der dritten Gestaltung eines Viskosimeters im Querschnitt;
- Fig. 11: ein Diagramm zur Illustration der Lage verschiedener Nukleotidsequenzen;
- Fig. 12: ein Diagramm mit Messwerten der Viskosität für verschiedene Proben.

### Ausführliche Beschreibung bevorzugter Ausführungsformen

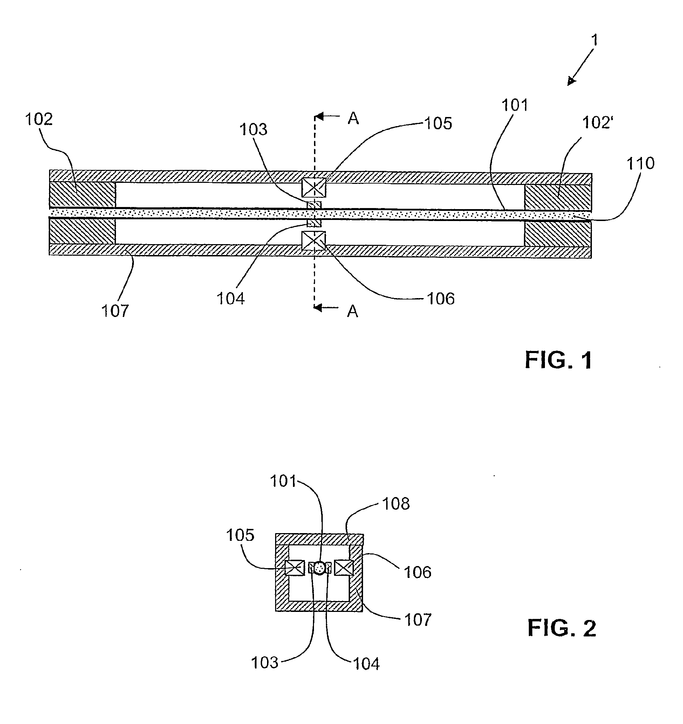

Die Fig. 1 zeigt in stark schematischer Darstellung eine erste Gestaltung einer Vorrichtung 1. Die Fig. 2 zeigt die Vorrichtung der Fig. 1 ebenfalls stark schematisch im Querschnitt in Richtung A-A gesehen. Gleiche Teile sind mit gleichen Bezugszeichen versehen.

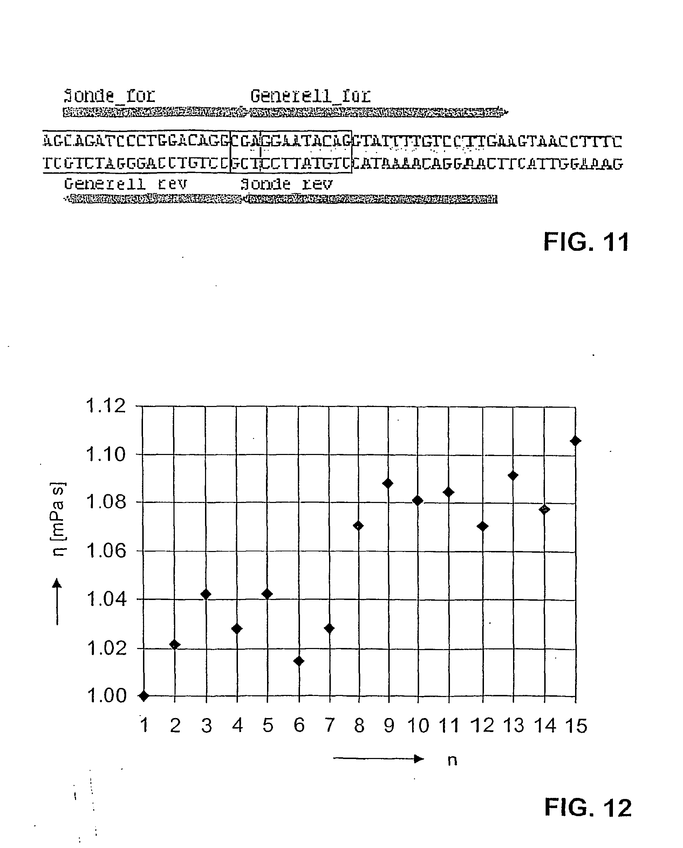

Ein Quarzglasrohr 101, das den Resonator bildet, ist im Bereich seiner beiden Enden in jeweils einer Inertialmasse 102, 102' befestigt. Die Inertialmassen sind mit einem Gehäuse 107 verbunden, z.B. durch Kleben. Beide Enden des Glasrohrs sind von aussen zugänglich, um ein flüssiges Reaktionsgemisch 110 in das Glasrohr einzuführen. Hierzu ragt das Glasrohr z.B. auf nicht dargestellte Weise an beiden Enden um einen gewissen Bereich aus den Inertialmassen heraus, und es sind geeignete Anschlüsse zum Zuführen des Reaktionsgemisches vorgesehen. Dies ist z.B. in der Fig. 12 der WO 95/24630 dargestellt. Im Betrieb dient somit das Innere des Glasrohrs als Kontaktoberfläche.

Auf der Mitte des Rohrs 101 sind diametral gegenüberliegend zwei Permanentmagnete 103, 104 aufgeklebt. Diesen benachbart sind im Gehäuse 107 zwei Elektromagnete 105, 106 befestigt. Die Elektromagnete 105, 106 bilden gemeinsam mit den Permanentmagneten 103, 104 einen elektromagnetischen Wandler zur Anregung von Torsionsschwingungen des Rohrs 101 und zur Detektion solcher Schwingungen. Die Funktionsweise solcher elektromagnetischer Wandler ist wohlbekannt. Insbesondere können die Rollen von Permanentmagneten und Elektromagneten vertauscht sein. Für die Funktionsweise und Ausgestaltung von solchen Wandlern wird ausdrücklich auf die WO 95/24630, Seite 10, Zeile 1 bis Seite 11, Zeile 9 Bezug genommen. Durch einen Stromfluss in den Elektromagneten 105, 106 wird über magnetische Kräfte ein Drehmoment auf die Permanentmagnete 103, 104 und dadurch auf den zentralen Bereich des Rohrs ausgeübt. Umgekehrt induziert eine Torsionsbewegung des Rohrs eine Spannung in den Elektromagneten 105, 106, die zur Detektion von Torsionsschwingungen dienen kann. Damit dient der Wandler abwechselnd sowohl als Aktuator zur Anregung als auch als Sensor zur Detektion. Selbstverständlich können aber auch getrennte Wandler zu diesen Zwecken vorgesehen werden.

Die Fig. 1 und 2 sind lediglich als schematische Darstellungen zu verstehen. So ist insbesondere aus Gründen der Darstellbarkeit der Durchmesser des Glasrohrs 101 stark vergrössert dargestellt. Tatsächlich beträgt der bevorzugte Aussendurchmesser des Glasrohrs weniger als 1 Millimeter, in einem konkret realisierten Beispiel nur 0,5 Millimeter. Die Wandstärke des Glasrohrs beträgt bevorzugt weniger als 0,1 Millimeter, im genannten Beispiel nur 0,05 Millimeter. Damit beträgt der Innendurchmesser im genannten Beispiel 0,4 Millimeter. Die Länge des Glasrohrs liegt bevorzugt zwischen etwa 30 Millimeter und 100 Millimeter, im genannten Beispiel ca. 80 Millimeter. Für dieses Beispiel beträgt das im Inneren des Glasrohrs vorhandene Probenraumvolumen ca. 10 mm³, also 10 Mikroliter. Mit einem solchen Viskosimeter lassen sich also sehr kleine Mengen an Reaktionsgemisch untersuchen.

Das Material des Glasrohrs ist bevorzugt ein Quarzglas. Dieses Material ist bevorzugt, da es eine sehr geringe Eigendämpfung und geringe Dichte aufweist, was einen hohen Q-Faktor und eine hohe Empfindlichkeit ermöglicht. Dank des niedrigen G-Moduls des Materials kann verhindert werden, dass die Grenzschicht zu klein wird. Bevorzugt liegt die Resonanzfrequenz der Grundschwingung im Bereich zwischen etwa 1 kHz und etwa 100 kHz; im genannten, konkret realisierten Beispiel betrug sie ca. 14,7 kHz. In diesem Beispiel war das Glasrohr ein kommerziell erhältliches Quarzglasrohr, wie es z.B. von VitroCom Inc., Mount Lakes, New Jersey (USA) erhältlich ist.

Durch das mit der Kontaktoberfläche in Berührung befindliche Reaktionsgemisch wird die Dämpfung des Resonators erhöht. Die Viskosität wird aus der gemessenen Dämpfung des Resonators bestimmt. Dies kann analytisch aus dem Zusammenhang von Viskosität und Dämpfung oder durch eine Kalibration erfolgen.

Die Vorrichtung wird dabei bevorzugt betrieben, wie es in der WO 95/24630 beschrieben ist. Hierzu sind die Elektromagnete 105, 106 des elektromagnetischen Wandlers mit einer Schaltung verbunden, wie sie in den Fig. 1 bis 8 sowie auf Seite 4, Zeile 1 bis Seite 9, Zeile 31 der WO 95/24630 dargestellt ist. Für den Aufbau der Schaltung zum Betrieb der Vorrichtung sowie für das Betriebsverfahren wird ausdrücklich auf diese Offenbarung Bezug genommen. Die Schaltung ist ein Beispiel einer Rückkopplungsschaltung, um den Resonator nahe seiner Eigenfrequenz zu stabilisieren. Die Dämpfung des Resonators wird bestimmt, indem die Frequenzverschiebung bei Änderung der Phase zwischen Anregungssignal im Wandler und detektiertem Signal im Wandler gemessen wird.

Die Vorrichtung der Fig. 1 und 2 wird bevorzugt so ausgestaltet, dass alle mit dem Reaktionsgemisch in Kontakt kommenden Teile, insbesondere das Glasrohr 101, leicht ausgewechselt werden können. Dazu wird das Glasrohr 101 in den Inertialmassen 102, 102' bevorzugt mit nicht näher dargestellten Befestigungsmitteln gehalten, die ein leichtes Lösen der Verbindung zwischen Glasrohr und Inertialmasse erlauben. Geeignet sind z.B. Klemmbuchsen aus Kunststoff oder Metall, wie sie im Stand der Technik wohlbekannt sind und die z.B. durch Verschrauben eine sichere Verbindung zwischen Glasrohr und Inertialmasse bewirken. Wichtig ist dabei, dass die Verbindung ausreichend fest ist, so dass der in den Befestigungsmitteln gehaltene Glasbereich tatsächlich nicht zu Torsionsschwingungen fähig ist.

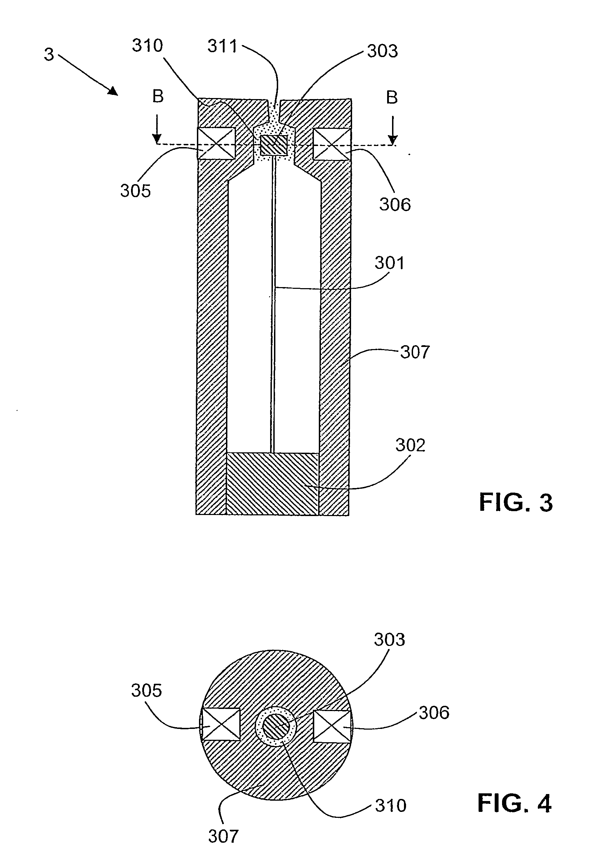

Die Fig. 3 zeigt eine schematische Darstellung einer zweiten Gestaltung einer Vorrichtung, mit der Ziffer 3 bezeichnet. Die Fig. 4 zeigt die Vorrichtung der Fig. 3 im Querschnitt in Blickrichtung B-B. Der Resonator umfasst erneut ein Quarzglasrohr 301, das mit einem Ende in einer Inertialmasse 302 befestigt ist. Am anderen Ende ist ein zylindrischer Kontaktkörper 303 aufgesetzt. Die Inertialmasse 302 ist in einem Gehäuse 307 befestigt. Dieses ist in der Umgebung des Kontaktkörpers so zylindrisch ausgebildet, dass es den Kontaktkörper entlang seiner Umfangsfläche mit einem konstanten Abstand umgibt. Gegenüber der Stirnfläche des Kontaktkörpers ist eine Öffnung 311 im Gehäuse vorhanden, durch die ein Reaktionsgemisch 310 in den vom Kontaktkörper 303 und dem Gehäuse 307 begrenzten Bereich eingebracht werden kann. Unterhalb des Kontaktkörpers weitet sich das Gehäuse innen stark auf. Ebenfalls weitet sich die Öffnung 311 von Kontaktkörper 303 weg auf. Diese Aufweitungen sowie die Abstände zwischen Kontaktkörper 303 und Gehäuse 307 sind so gewählt, dass das Reaktionsgemisch durch Kapillarkräfte im vorgesehenen Bereich gehalten wird. Durch geeignete Wahl der Grösse des Kontaktkörpers und der Abstände kann zudem die Menge des Reaktionsgemisches sehr stark reduziert werden.

In der dargestellten Vorrichtung ist der Kontaktkörper 303 als ein Permanentmagnet ausgebildet, dessen Magnetfeld im wesentlichen senkrecht zur langen Achse des Rohres 301 verläuft. Zum Schutz vor Korrosion durch das Reaktionsgemisch kann der Magnet mit einem chemischen inerten Überzug versehen sein. Dieser Permanentmagnet ist zu magnetischen Wechselwirkungen mit zwei Elektromagneten 305, 306 geeignet. Kontaktkörper 303 und Elektromagnete 305, 306 bilden demnach zusammen einen elektromagnetischen Wandler, und der Kontaktkörper ist selbst Teil dieses Wandlers.

In einer konkreten Gestaltung wird der Kontaktkörper z.B. von einem Sm-Co-Permanentmagneten gebildet, der an seiner Oberfläche mit bekannten Methoden hydrophilisiert wurde, um eine gute Benetzung sicherzustellen.

Durch Anregung des Wandlers mit elektrischen Signalen passender Frequenz wird das Rohr 301 zu Torsionsschwingungen angeregt, bevorzugt in der Grundschwingung. Die Schwingungen werden vom selben Wandler als Sensor detektiert. Das Viskosimeter der Fig. 3 wird dabei im wesentlichen wie dasjenige der Fig. 1 betrieben; das oben hierzu Gesagte gilt dementsprechend analog. Die Resonanzfrequenz des Resonators liegt wiederum bevorzugt im Bereich zwischen 1 und 100 kHz.

Die Fig. 3 und 4 sind wiederum nur als schematische Darstellungen zu verstehen, in denen insbesondere die Grössenverhältnisse nicht denjenigen der Realität entsprechen müssen. Bezüglich der Überlegungen zu den Abmessungen des Glasrohrs gilt das zu Fig. 1 und 2 Gesagte. Der Abstand zwischen dem Kontaktkörper und dem Gehäuse ist mindestens grösser als die Dicke der Grenzschicht des Reaktionsgemisches zu wählen, die sich bei der Torsionsschwingung des Resonators ausbildet. Bezüglich weiterer Abmessungen ist dem Fachmann geläufig, wie diese zu wählen sind, um je nach Material des Kontaktkörpers und des Gehäuses sowie der Zusammensetzung des Reaktionsgemisches ausreichende Kapillarkräfte zu erreichen. In einer konkreten Ausgestaltung weist z.B. der Kontaktkörper einen Durchmesser von 2,0 Millimeter und eine Länge von 0,7 Millimeter auf. Der Innendurchmesser des den Kontaktkörper umgebenden Gehäuses beträgt 3,0 Millimeter, d.h. der Spalt zwischen Kontaktkörper 303 und Gehäuse 307 weist eine Breite von 0,5 Millimeter auf. Das gesamte Probenvolumen in einem solchen Viskosimeter beträgt etwa 15 Mikroliter. Selbstverständlich können diese Abmessungen, abhängig vom konkreten Einsatzgebiet des Viskosimeters, in einem weiten Bereich variieren. Ein wichtiger Gesichtspunkt bei der Wahl der Abmessungen des Kontaktkörpers ist die Resonanzfrequenz des Resonators, die umso niedriger liegt, je grösser die Masse des Kontaktkörpers 303 ist.

Das Viskosimeter der Fig. 3 eignet sich aufgrund seines einfachen Aufbaus besonders auch als Einweg-Viskosimeter, dessen mit dem Reaktionsgemisch in Kontakt tretenden Teile nach Gebrauch einfach weggeworfen werden können. In einem solchen Fall sind die Elektromagneten 305, 306 des Wandlers leicht abnehmbar am Gehäuse 307 befestigt. Gehäuse 307, Inertialmasse 302, Rohr 301 und Kontaktkörper 303 bilden eine Einweg-Einheit, die nach Gebrauch weggeworfen und durch eine neue Einheit ersetzt werden. Die Elektromagneten 305, 306 wie auch die nicht dargestellte Elektronik zur Ansteuerung werden dagegen weiterverwendet. Das Gehäuse kann in einem solchen Fall kostengünstig aus einem Kunststoff, z.B. Polypropylen, hergestellt werden, insbesondere in einem (Präzisions-) Spritzgussverfahren. Dasselbe gilt für die Inertialmasse. Die Elektromagnete können auch in einem separaten Gehäuse, z.B. einem Haltering, gehalten sein, wobei das Viskosimeter-Gehäuse 307 in dieses z.B. einschiebbar ausgestaltet ist.

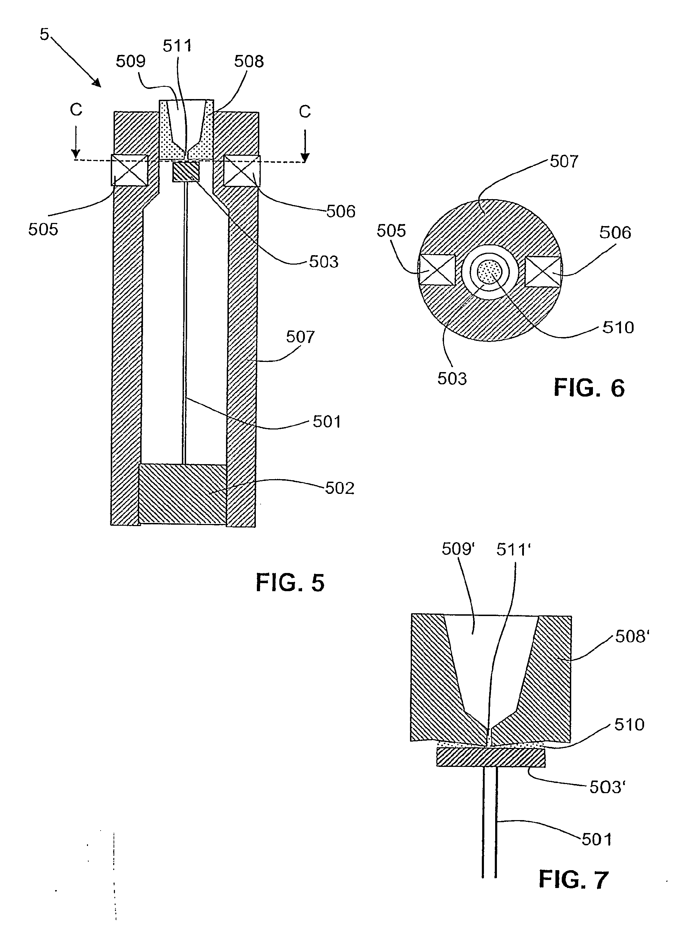

Die Fig. 5 zeigt eine schematische Darstellung einer dritten Gestaltung der Vorrichtung, mit der Ziffer 5 bezeichnet. Diese Vorrichtung ist in der Fig. 6 im Querschnitt in der Blickrichtung C-C dargestellt. Wiederum umfasst der Resonator ein Glasrohr 501, welches an einem Ende mit einer Inertialmasse 502 verbunden ist. Die Inertialmasse ist in einem Gehäuse 507 eingesetzt. Am anderen Ende des Glasrohres 501 ist wiederum ein Kontaktkörper 503 angebracht. Ebenso wie in der Fig. 3 ist dieser als Permanentmagnet ausgebildet und wechselwirkt magnetisch mit Elektromagneten 505, 506. Er ist also Teil eines elektromagnetischen Wandlers.

Gegenüber der Stirnfläche des Kontaktkörpers 503 ist ein Dosierelement 508 in das Gehäuse 507 eingeschraubt. Dieses weist einen trichterförmigen Dosierbereich 509 zur Aufnahme eines Reaktionsgemisches 510 auf, der nach unten hin in eine Öffnung 511 mündet. Durch diese Öffnung gelangt das Reaktionsgemisch auf die Kontaktoberfläche, die durch die Stirnfläche des Kontaktkörpers 503 gebildet wird. Durch Kapillarkräfte wird das Reaktionsgemisch auf dieser Fläche gehalten. Der Probenraum ist damit auf den Bereich zwischen der Stirnfläche des Kontaktkörpers 503 und dem Dosierelement 508 beschränkt. Der Abstand zwischen Kontaktkörper und Dosierelement kann z.B. mittels eines nicht dargestellten Feingewindes auf der zylindrischen Umfangsfläche des Dosierelements 508, das mit einem entsprechenden Innengewinde des Gehäuses 507 zusammenwirkt, variiert werden. Damit kann das Reaktionsgemisch auf eine dünne Schicht begrenzt werden, deren Dicke in der Grössenordnung der Grenzschicht eingestellt werden kann, die sich ausbildet, wenn der Resonator Torsionsschwingungen ausführt.

Die Fig. 7 zeigt einen Ausschnitt aus einem ähnlichen Viskosimeter, welches gemäss den Prinzipien der Fig. 5 aufgebaut ist. Nur ein Teil des Glasrohrs 501, der Kontaktkörper 503' und das Dosierelement 508' mit Dosierbereich 509' und Öffnung 511' sind, zusammen mit dem Reaktionsgemisch 510, dargestellt. Während die Stirnfläche des Kontaktkörpers 503' eben ist, weist das Dosierelement 508' im Bereich gegenüber der Stirnfläche eine konische Form auf. Dadurch weitet sich der Abstand zwischen dem Kontaktkörper 503' und dem Dosierelement 508' radial auf. Diese Konfiguration stellt einerseits eine wirksame Begrenzung des Reaktionsgemisches durch Kapillarkräfte auf den gewünschten Bereich sicher. Andererseits stellt diese Konfiguration sicher, dass über das gesamte Reaktionsgemisch eine im wesentlichen räumlich uniforme Schergeschwindigkeit (uniformer Geschwindigkeitsgradient entlang der axialen Richtung) erreicht wird. Mit dieser Konfiguration können sehr kleine Probenvolumina erreicht werden, insbesondere im Bereich von wenigen Mikrolitern.

Zum Betrieb einer Vorrichtung gemäss den Fig. 5 bis 7 gilt wiederum das zu den Fig. 3 und 4 Gesagte.

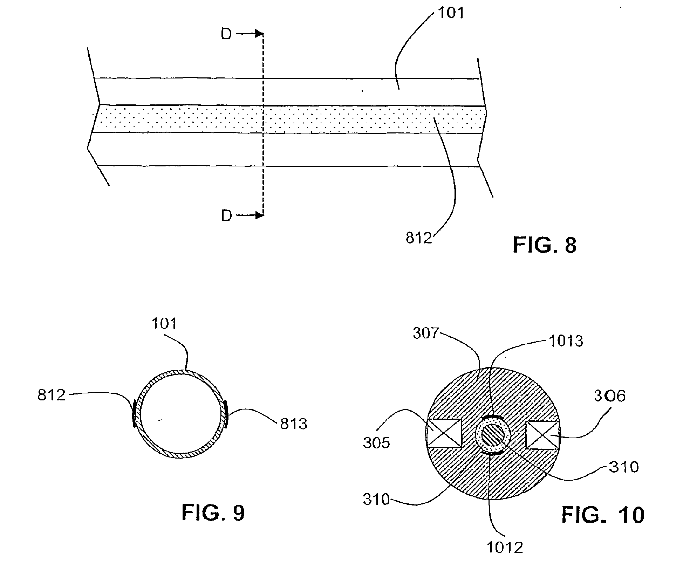

Die Viskosimeter können mit Einrichtungen zum Temperieren (Heizen und/oder Kühlen) des Reaktionsgemisches versehen sein. Die Fig. 8 zeigt als Beispiel schematisch und stark vergrössert einen Teil eines Glasrohrs 101 aus einem Viskosimeter gemäss der Fig. 1. Die Fig. 9 stellt das Glasrohr im Querschnitt in Blickrichtung D-D dar. Auf dem Glasrohr 101 sind zwei Metallstreifen 812, 813 aufgedampft, die als Heizdrähte wirken. Hiermit lässt sich das im Innern des Glasrohres 101 befindliche Reaktionsgemisch leicht aufheizen. Anstelle einer in Axialrichtung verlaufenden Schicht kann diese auch z.B. schraubenförmig um das Glasrohr verlaufen.

Alternativ oder zusätzlich kann in der Umgebung des Probenraums ein Peltierelement, wie es wohlbekannt ist, angeordnet werden. Damit wird es möglich, das Reaktionsgemisch wahlweise zu heizen oder zu kühlen. Ein geeignetes Peltierelement kann z.B. direkt auf dem Glasrohr aufgebracht (z.B. aufgeklebt) werden, oder es kann auf dem Glasrohr gebildet werden, indem zwei unterschiedliche Metalle aufgedampft werden. In Abhängigkeit von der Stromrichtung kann ein Peltierelement sowohl als Heizelement als auch als Kühlelement dienen.

In einer bevorzugten Gestaltung ist das Temperierelement gleichzeitig Teil des elektromagnetischen Wandlers, d.h. auf die Elektronen im Metall des Temperierelements wirkt in einem Magnetfeld eine Lorentzkraft, die zur Anregung des Resonators und/oder zur Detektion der Schwingungen des Resonators herangezogen wird. Zu diesem Zweck können zusätzlich zum Temperierelement auch weitere Metallstreifen aufgedampft oder in anderer Weise aufgebracht sein, z.B. in der Form von Spulen. Das als Teil des Wandler wirkende Temperierelement und/oder die zusätzlichen Metallstreifen können in einer ersten Betriebsart mit Gleichstrom betrieben werden. Im Betrieb wirken sie dann mit einem alternierenden Magnetfeld zusammen, das von den Elektromagneten 105, 106 erzeugt wird, um den Resonator anzuregen. Alternativ können sie auch mit Wechselstrom betrieben werden. In diesem Falle können die Spulen 105, 106 durch Permanentmagneten ersetzt werden, und sowohl die Anregung als auch die Schwingungsdetektion erfolgt vollständig mittels des Temperierelements bzw. der zusätzlichen Metallstreifen statt mittels der Spulen 105, 106.

Insbesondere im Falle eines Peltierelements als Teil des Wandlers wird dieses zu Zwecken der Anregung vorteilhaft mit Wechselstrom betrieben, so dass keine nennenswerte Kühl- oder Heizwirkung erfolgt, da sich Kühl- und Heizeffekt über eine Periode des Wechselstroms weitgehend aufheben.

Die auf dem Glasrohr 101 vorhandenen Metallbereiche können in bekannter Weise an elektrische Zuleitungen angeschlossen werden. Dazu sind z.B. nahe der Enden des Glasrohrs Kontaktfelder ausgebildet. Nach dem Einsetzen des Glasrohrs in die Inertialmassen stehen diese Kontaktfelder mit Kontakten für die elektrischen Zuleitungen in Berührung, welche z.B. in der Bohrung der Inertialmassen oder an anderer geeigneter Stelle bereitgestellt werden.

Eine solche Ausgestaltung ist besonders dann vorteilhaft, wenn das Glasrohr als Einweg-Glasrohr ausgestaltet ist, also dazu ausgebildet ist, nach Gebrauch entfernt und ersetzt zu werden. Insbesondere können die Permanentmagnete 103, 104 entfallen, was zu einer deutlichen Kosteneinsparung führt.

Als ein weiteres Beispiel zeigt die Fig. 10 eine Variante des Viskosimeters der Fig. 4, bei der sich an der den Kontaktkörper 303 umgebenden Innenfläche des Gehäuses 307 zwei Heizelemente 1012, 1013 befinden. Diese können z.B. aus stromdurchflossenen Drahtstücken gebildet sein oder in anderer bekannter Weise ausgebildet sein.

Es können auch mehrere Temperierelemente vorhanden sein, die z.B. die Erzeugung von Temperaturgradienten ermöglichen. Vorzugsweise sind ausserdem geeignete Temperaturfühler vorhanden, wie sie im Stand der Technik wohlbekannt sind (z.B. Pt-100-Fühler usw.)

Das Vorhandensein eines oder mehrerer Temperierelemente ist vor allem dann besonders nützlich, wenn eine Reaktion direkt im Viskosimeter durchgeführt werden soll, birgt aber auch sonst Vorteile, da die Viskosität in der Regel stark temperaturabhängig ist und eine Temperierung die Reproduzierbarkeit der Messung verbessern kann. So kann bei Vorhandensein eines Kühlelements das Reaktionsgemisch z.B. auf eine Temperatur nahe dem Erstarrungspunkt des Reaktionsgemisches abgekühlt werden, z.B. in den Bereich zwischen 0 °C und 10 °C bei verdünnten wässrigen Lösungen, was in der Regel eine signifikante Viskositätszunahme bewirkt, und dort stabilisiert werden. Häufig wird eine Stabilisierung im Bereich von z.B. ±0,1 °C erforderlich sein, was sich mit den genannten Temperierelementen schneller und leichter erreichen lässt als mit einer Temperierung des gesamten Viskosimeters.

Besonders interessant ist eine Temperierung, wenn eine Amplifikationsreaktion von Nukleinsäurefragmenten durchgeführt wird, insbesondere eine PCR oder LCR, die häufige Temperaturänderungen (Temperaturzyklen) erfordern. Durch die Temperierung kann die Reaktion direkt im Probenraum des Viskosimeters stattfinden, und es kann die Amplifikation direkt an geeigneten Punkten der Temperaturzyklen anhand der Viskositätsänderung verfolgt werden. Bei einem Verfahren zur Mutationsdetektion mittels allelspezifischer Amplifikation kann die Reaktion z.B. unmittelbar beendet werden, sobald eine Viskositätsänderung eintritt, die das Vorliegen der Mutation anzeigt. Ohne eine Temperierung des Viskosimeters wird die Amplifikation in einer getrennten Reaktionsvorrichtung für den Amplifikationsprozess zunächst für eine feste Zyklenzahl durchgeführt. Die Probe muss dann der Reaktionsvorrichtung entnommen werden und dann erst ins Viskosimeter transferiert werden. Es ist offensichtlich, dass dies mit höherem Zeit- und Arbeitsaufwand verbunden ist.

Der Einsatz des erfindungsgemässen Verfahrens in einer Amplifikationsreaktion von Nukleinsäuren soll nun anhand einer Anwendung zur Mutationsdetektion beispielhaft erläutert werden.

### Anwendung: Mutationsdetektion

Zur Mutationsdetektion auf Nukleinsäuren (DNA und RNA) werden heute üblicherweise verschiedene Methoden herangezogen. Mutationen auf dem Erbgut sind unter anderem das Fehlen einzelner Basenpaare (single nucleotide polymorphisms SNPs), das Fehlen einer ganzen Sequenzeinheit (Deletion) oder das Vorhandensein einer überzähligen Erbinformation (Insertion). Als Beispiele für den Nachweis solcher Erbgutveränderungen seien die Gelelektrophorese mit Restriktionsenzymen erwähnt, welche als Anfärbemethode Ethidiumbromid verwendet. Ein moderneres Beispiel ist die Echt-Zeit-PCR (Realtime PCR) mit Fluorophoren zur Visualisierung der Reaktion. Diese Prozesse sind kostspielig, zeitintensiv und relativ teuer.

Das erfindungsgemässe Verfahren stellt eine kostengünstige Alternative dar, mit der die Ermittlung einer Mutation auf dem Erbgut möglich wird. Vorteilhaft läuft das Verfahren in folgenden Phasen ab:
- Isolation der Nukleinsäuren;
- Amplifikation der zu untersuchenden Sequenz, z.B. mittels Polymerase-Kettenreaktion (PCR);
- Verknüpfung der in der Amplifikationsreaktion entstandenen Fragmente mittels einer Ligase-Kettenreaktion (LCR) mit speziell als Linker ausgebildeten Sondenmolekülen (allelspezifisch);
- Ermittlung der Viskosität des Reaktionsprodukts.

Die Viskositätszunahme bei einem chemischen Prozess wie der PCR ist im Prinzip theoretisch berechenbar. Es werden dabei Nukleotide in längere, doppelsträngige Produkte umgewandelt, so dass die Moleküle grösser werden und Wasser freigesetzt wird, das vorher die Nukleotide umgeben hat. Die Viskosität wird sich dabei verändern, da Polymere synthetisiert werden. Die in der Reaktion entstehenden sogenannten Templates erreichen häufig eine Grösse von 250 bis 500mer.

Allerdings reicht der Amplifikationsprozess häufig allein nicht aus, um eine signifikante Viskositätszunahme zu messen. Aus diesem Grunde werden die bei der Amplifikation entstehenden Moleküle vorzugsweise untereinander verbunden (lineare Verknüpfung und/oder Vernetzung). Dieser Prozess wird anschliessend an die Amplifikation oder gekoppelt an diese durchgeführt. Die Verknüpfung wird dabei bevorzugt mit einer LCR unter Einbau von geeigneten Linkermolekülen durchgeführt.

### Beispiel: Detektion der "Faktor V Leiden"-Mutation

Die Mutation für den Faktor V Leiden ist eine einzelne Punktmutation (G statt A) in der menschlichen Gensequenz, die dazu führt, dass im von der Sequenz kodierten Protein (ein Gerinnungsfaktor) an Position 506 ein Glutaminrest durch einen Argininrest ersetzt wird. Weitere Informationen über diese Mutation finden sich z.B. in R.M. Bertina et al., "Mutation in Blood Coagulation Factor V Associated with Resistance to Activated Protein C", Nature 369, 64-67 (1994). Die gesamte Nukleotidsequenz des Faktor V-Gens ist beschrieben in: R.J Jenny et al., "Complete cDNA and Derived Amino Acid Sequence of Human Factor V", Proc. Nat. Acad. USA 84, 4846-4850 (1987). In Individuen mit der Mutation ist die Thrombosegefahr gegenüber Individuen des Wildtyps markant erhöht, weshalb diese Mutation Gegenstand eines häufig durchgeführten Labortests in klinischen Genetiklabors ist.

Im üblicherweise durchgeführten Verfahren zur Identifikation der Mutation wird das Gensegment mittels PCR amplifiziert. Das Amplifikationsprodukt wird mit einer Restriktions-Endonuklease verdaut, welche nur die Wildtypsequenz schneidet, jedoch nicht die Mutante. Wildtyp und Mutante lassen sich dann durch Gelelektrophorese unterscheiden. Dieses Verfahren ist zeitaufwändig und benötigt eine grosse Zahl manueller Schritte.

Alternativ wurde in US 6,174,670 vorgeschlagen, den PCR-Vorgang mittels Fluoreszenz zu überwachen. Dieses Verfahren ist zwar wesentlicher schneller, wegen der Notwendigkeit optischer Komponenten und des Einsatzes von Fluoreszenzmarkern aber relativ teuer.

Es wird deshalb im Folgenden demonstriert, dass sich die Mutation des Leiden V-Gens auch sehr kostengünstig, einfach und schnell mit dynamischer Viskosimetrie untersuchen lässt.

Im Folgenden wird als "Mutationsstelle" diejenige Nukleotidposition im Gen des Faktors V bezeichnet, an der eine Guaninbase im Wildtyp durch eine Adeninbase in der Mutante ersetzt ist.

Es wurden vorab die in Tabelle 1 angegebenen DNA-Oligonukleotide synthetisiert (TIB-MOLBIOL, Berlin). Fig. 11 illustriert dabei, wie diese Oligonukleotide mit der Sequenz des Faktor-V-Gens korrespondieren. In der Fig. 11 ist zentral ein Ausschnitt des Doppelstrangs des Faktor-V-Gens in der Umgebung der Mutationsstelle dargestellt. Die Oligonukleotide sind dazu ausgebildet, an den angegebenen Stellen an die Einzelstränge zu hybridisieren. Hierbei steht "Sonde_for" für entweder "Wildtyp_for" oder "Mutation_for", entsprechend steht "Sonde_rev" für entweder "Wildtyp_rev" oder "Mutation_rev". In einer "normalen" LCR dient das Oligonukleotid "Sonde_for" zur Verbindung mit dem Oligonukleotid "Generell_for", das am 5'-Ende phosphoryliert ist, analog das Oligonukleotid "Sonde_rev" zur Verbindung mit dem 5'-phosphorylierten Oligonukleotid "Generell_rev". Die Moleküle "WT_Linker_for", "WT_Linker_rev", "Mut_Linker_for" und "Mut_Linker_for" entstehen durch eine Verbindung dieser Moleküle via einen Linker aus (vorzugsweise) neun T-Basen und sind dazu ausgebildet, mit ihren 5'-seitigen Abschnitten auf ein erstes Nukleotid und ihren 3'-seitigen Abschnitten auf ein anderes Nukleotid zu hybridisieren.

**Tabelle 1: DNA-Oligonukleotide zur Analyse der Mutation Faktor V Leiden.**

| Name | Sequenz (5' → 3') | ID | Tm |
|---|---|---|---|
| Primer_for | TAATCTGTAA GAGCAGATCC | SEQ ID NO: 1 | |
| Primer_rev | TGTTATCACA CTGGTGCTAA | SEQ ID NO: 2 | |
| Wildtyp_for | AGATCCCTGG ACAGGCG | SEQ ID NO: 3 | 56,1°C |
| Mutation_für | CAGATCCCTG GACAGGCA | SEQ ID NO: 4 | 56,4°C |
| Generell_for | p-AGGAATACAG GTATTTTGTC CTTGA | SEQ ID NO: 5 | 55,6°C |
| Wildtyp_rev | CAAGGACAAA ATACCTGTAT TCCTC | SEQ ID NO: 6 | 55,6°C |
| Mutation_rev | CAAGGACAAA ATACCTGTAT TCCTT | SEQ ID NO: 7 | 55,7°C |
| Generell_rev | p-GCCTGTCCAG GGATCTG | SEQ ID NO: 8 | 53,6°C |
| WT_Linker_for | p-AGGAATACAG GTATTTTGTC CTTGATTTTT TTTTAGATCC CTGGACAGGC G | SEQ ID NO: 9 | |
| WT_Linker_rev | p-GCCTGTCCAG GGATCTGTTT TTTTTTCAAG GACAAAATAC CTGTATTCCT C | SEQ ID NO: 10 | |
| Mut_Linker_for | p-AGGAATACAG GTATTTTGTC CTTGATTTTT TTTTAGATCC CTGGACAGGC A | SEQ ID NO: 11 | |
| Mut_Linker_rev | p-GCCTGTCCAG GGATCTGTTT TTTTTTCAAG GACAAAATAC CTGTATTCCT T | SEQ ID NO: 12 | |

Hierbei gibt der Buchstabe "p" an, dass die jeweilige Base am betreffenden 5'- bzw. 3'-Ende phosphoryliert ist. Tm gibt den nach der Nearest-Neighbor-Methode berechneten Schmelzpunkt an. Diese Oligonukleotide dienten als Primer bzw. Sonden für die nachfolgend beschriebenen Reaktionen.

Die Untersuchung auf Vorliegen der Mutation umfasste die Bereitstellung einer Probe humaner DNA, welche die Mutationsstelle umfasste, eine Amplifikation mittels PCR, gefolgt von einer "normalen" LCR oder einer allelspezifischen und speziell adaptierten LCR, schliesslich gefolgt von einer Viskositätsmessung.

Humane genomische DNA (Ausgangs-DNA) wurde aus Vollblut nach Verfahren isoliert, wie sie im Fachgebiet wohlbekannt sind, siehe z.B. J. Sambrook et al., "Molecular Cloning: A Laboratory Manual" (2nd edition, 1989), Kapitel 9.

Mit dem Nukleinsäure-Isolat wurde zunächst eine PCR zur (unspezifischen) Amplifikation der Ausgangs-DNA wie folgt durchgeführt: Als Primer dienten die oben genannten Oligonukleotide "Primer_for" (SEQ ID NO 1) und "Primer_rev" (SEQ ID NO 2). Es wurde eine PCR nach folgendem Protokoll durchgeführt:
Denaturation: 95 °C, 1 s
Annealing: 55 °C, 10 s
Elongation: 72 °C, 15 s
Cyclen: 20
Konzentrationen: 0.5 Mikromol jedes Oligonukleotids
Taq-Polymerase 5 U / Mikroliter, insgesamt 3 Mikroliter
MgCl₂: 3 mM
dNTPs: 50 Mikromol

Nur optional wurde anschliessend an die PCR eine "normale" LCR durchgeführt. Die LCR wurde wie folgt durchgeführt: "Ampligase 1X Reaction Buffer" von Epicentre Technologies (20 mM Tris-HCl (pH 8.3), 25 mM KCI, 10 mM MgCl₂, 0,5 mM NAD, und 0.01% Triton X-100) wurde bereitgestellt. Zu 50 Mikroliter Reaktionspuffer wurde zugegeben: Je 50 nmol der genannten Sonden "Sonde_for" (SEQ ID NO 3 oder 4), "Generell_for" (SEQ ID NO 5), "Sonde_rev" (SEQ ID NO 6 oder 7) und "Generell_rev" (SEQ ID NO 8), 1.5 - 5 U Ampligase (Epicentre Technologies). Die Konzentration der Ampligase ist abhängig von der Art der eingesetzten Ampligase. 30 Sekunden Inkubation bei 95 °C, gefolgt von 30 Sekunden Inkubation bei 50 °C während 45 Sekunden; 35 Zyklen.

In diesem Falle findet eine normale allelspezifische LCR statt, bei der die Ligase jeweils zwei "forward" oder "reverse"-Sonden verknüpft, die auf demselben Nukleotid anhybridisiert sind. Es erfolgt also keine weitergehende Verknüpfung (Verkettung oder Vernetzung) der entstehenden Nukleotide.

Alternativ wurde eine speziell adaptierte LCR durchgeführt. Die LCR war dabei so ausgestaltet, dass gleichzeitig eine Vernetzung der entstehenden Nukleinsäurefragmente stattfindet. Hierzu dienten die Sonden "WT_Linker_for" (SEQ ID NO 9) und "WT_Linker_rev" (SEQ ID NO 10) als spezielle Überbrückungsmoleküle, welche zwischen den beiden Enden je eines von der Polymerase synthetisierten PCR-Produkte binden. So umfasst z.B. die Sonde "WT_Linker_for" (SEQ ID NO 9) sowohl die Sequenz der Sonde "Generell_for" (SEQ ID NO 5) als auch die Sequenz "Wildtyp_for" (SEQ ID NO 3). Diese sind mittels eines Linkerbereich aus 9 T-Basen verbunden, wobei die Zahl der Basen auch anders gewählt werden kann. Vernünftig scheinen zwischen 5 und 20 Basen im Linkerbereich zu sein, bevorzugt 5 bis 10.

Während der LCR hybridisiert nun der 5'-Bereich der Sonde "WT_Linker_for" (SEQ ID NO 9) an den entsprechenden Bereich eines ersten Nukleotids, während der 3'-Bereich an den entsprechenden Bereich eines zweiten Nukleotids hybridisiert. Falls auf diesem zweiten Nukleotid das 5'-Ende eines weiteren Sondenmoleküls anhybridisiert ist, werden die beiden Sondenmoleküle durch die Ligase verbunden. Auf diese Weise entsteht ein längerkettiges Polymer. Die Entstehung dieses Polymers führt zu einem markanten Viskositätsanstieg.

Allerdings wird die Ligase-Verknüpfung nur dann erfolgen, wenn die Sequenz der Sonde am 3'-Ende exakt mit der Sequenz des Nukleotids korrespondiert. Ein einzelner Basenaustausch am 3'-Ende wird daher die LCR inhibieren. Auf diese Weise wird die LCR zudem allelspezifisch.

Die vorstehende Erklärung bezog sich auf die Sonde "WT_Linker_for", gilt aber analog auch für die Sonde "WT_Linker_rev". Die Reaktion wird simultan auf dem (+) und (-)-DNA-Strang durchgeführt, in Anwesenheit beider Sonden.

Insgesamt handelt es sich also um eine allelspezifische LCR, die gleichzeitig zu einer Polymerisation von Sondenmolekülen führt.

Die adaptierte LCR wurde wie folgt durchgeführt: "Ampligase 1X Reaction Buffer" von Epicentre Technologies; 50 Mikroliter Reaktionspuffer, je 50 nmol der genannten Sonden "WT_Linker_for", "WT_Linker_rev", 1.5 - 5 U Ampligase (Epicentre Technologies). Die Konzentration der Ampligase ist abhängig von der Art der eingesetzten Ampligase. 30 Sekunden Inkubation bei 95 °C, gefolgt von 30 Sekunden Inkubation bei 50 °C während 45 Sekunden; 35 Zyklen.

Das Produkt wurde anschliessend in einem dynamischen Viskosimeter untersucht, wie es in Fig. 1 dargestellt ist (Quarzglasrohr als Resonator, Länge 80 mm, Aussendurchmesser 0,5 mm, Wandstärke 0,05 mm). Dabei wurde die Probe auf 10 °C abgekühlt und die Temperatur in einem Bereich von ±0,1 °C stabilisiert.

Es wurde die Viskosität der folgenden Proben bestimmt.

Referenzmessungen:
- Probe 1: H₂O (PCR grade)
- Probe 6: Pufferlösung für PCR (ohne weitere Zusätze)
- Probe 7: Pufferlösung für LCR (ohne weitere Zusätze)

Auf diesen Proben wurde weder eine PCR noch eine LCR ausgeführt.

### Kontrollmessungen:

- Probe 9: LCR-Puffer, dem eine zuvor mit Linkermolekülen polymerisierte wt-Probe aus einem anderen System beigegeben wurde
- Probe 10: PCR-Puffer mit Farbstoff SYBR Green, dem eine zuvor mit Linkermolekülen polymerisierte wt-Probe aus einem anderen System beigegeben wurde

Auch auf diesen Proben wurde keine weitere PCR oder LCR ausgeführt.

Die weiteren Proben waren wie in Tabelle 2 dargestellt zusammengesetzt. Dabei bedeutet:
- Target-DNA: Es war Target-DNA wie folgt vorhanden: wt = Wildtyp (gesund), mut = homozygot mutiert.
- PCR: Falls ja: Es wurde eine PCR vor der nachfolgenden LCR durchgeführt.
- LCR konv.: Falls ja: es wurde eine konventionelle LCR mit den Sonden der angegebenen SEQ ID NO aus Tabelle 1 durchgeführt, d.h. mit Sonden ohne Linker.
- LCR-Linker: Falls ja: es wurde eine adaptierte LCR mit den Linker-Sonden der angegebenen SEQ ID NO aus Tabelle 1 durchgeführt, d.h. eine LCR mit gleichzeitiger Polymerisation der Sondenmoleküle.

**Tabelle 2: Proben für Viskositätsmessungen**

| **Nummern** | **Target-DNA** | **PCR** | **LCR konv. (Sonden)** | **LCR-Linker (Sonden)** |
|---|---|---|---|---|
| **1** | H₂O PCR grade | | | |
| **2** | wt | nein | ja (3, 5, 6, 8) | nein |
| **3** | wt | nein | ja (3, 5, 6, 8) | nein |
| **4** | mut | ja | ja (3, 5, 6, 8) | nein |
| **5** | wt | ja | ja (4, 5, 7, 8) | nein |
| **6** | Puffer für PCR, ohne andere Zusätze | | | |
| **7** | Puffer für LCR, ohne andere Zusätze | | | |
| **8** | wt | ja | nein | ja, mut-Linker (11, 12) |
| **9** | LCR-Puffer mit gelinkter wt-Probe aus einem anderen System | | | |
| **10** | PCR-Puffer mit SYBR Green, mit gelinkter wt-Probe aus einem anderen System | | | |
| **11** | mut | ja | nein | ja, mut-Linker (11, 12) |
| **12** | wt | nein | nein | ja, wt-Linker (9, 10) |
| **13** | mut | ja | nein | ja, mut-Linker (11, 12) |
| **14** | mut | nein | nein | ja, mut-Linker (11, 12) |
| **15** | wt | ja | nein | ja, wt-Linker (9, 10) |

Die Ergebnisse sind in der Fig. 12 dargestellt. Diese stellt das Ergebnis von Viskositätsmessungen an den in Tabelle 2 angegebenen Proben graphisch dar.

Hierbei bezeichnet das Symbol n die fortlaufende Nummer der Messung, η die Viskosität. Es wurden zudem noch weitere, nicht dargestellte Kontrollmessungen ausgeführt.

Die Ergebnisse zeigten, dass eine Mutationsdetektion mittels dynamischer Viskosimetrie möglich ist. Dabei tritt eine Viskositätserhöhung nur auf, wenn die Amplifikation genügend Nukleinsäurefragmente als Produkt hervorgebracht hat, die bei der anschliessenden adaptierten LCR als Templates dienen können. Somit kann aus An- oder Abwesenheit einer Viskositätsänderung bei der Ligase-Verknüpfung auf das Vorliegen oder die Abwesenheit der Mutation geschlossen werden.

Es soll betont werden, dass die Erfindung keineswegs auf die vorstehenden Beispiele beschränkt ist. So sind in dem Verfahren zur Ermittlung des Reaktionszustands einer Amplifikationsreaktion insbesondere vielfältige Änderung des Chemiedesigns möglich.

So ist für das Design des Verfahrens die Anzahl bereits vorhandener Nukleinsäuren entscheidend. Ist eine genügende Anzahl Moleküle vorhanden, kann z.B. auf eine PCR-Amplifikation verzichtet werden und gleich die LCR durchgeführt werden. Beispielsweise kann zur Detektion von DNA aus Bakteriensuspensionen alternativ zur PCR mit gekoppelter LCR auch eine LCR alleine, ohne PCR, durchgeführt werden. Ähnlich kann für die Detektion von DNA aus Thymus nur eine PCR oder nur eine LCR durchgeführt werden, wenn die Nukleinsäuremenge derart gross ist, dass eine Viskositätsänderung durch eines dieser Verfahren alleine messbar wird.

Die speziell adaptierte LCR, wie sie hier beschrieben wurde, ist zwar vorteilhaft, aber nicht unbedingt in allen Situationen essentiell. So kann unter Umständen allein die Viskositätssteigerung aufgrund einer PCR oder einer konventionellen LCR zur Detektion genügen. Bei genügend hoher Zahl an Nukleinsäuresequenzen könnte insbesondere allein mittels allelspezifischer Amplifikation eine Produktzunahme aufgrund einer Viskositätssteigerung detektiert werden, als eine Form von "Echtzeit-PCR".

Um eine Punktmutation (SNP) anhand einer Viskositätszunahme detektieren zu können, kann auch der PCR-Prozess in Abhängigkeit zur Mutation gebracht werden, indem die Amplifikation nur stattfindet, wenn die Mutation vorhanden ist. In einem solchen Fall könnten dann bei anschliessender LCR auch nur die amplifizierten Sequenzen ligiert werden, also nur diejenigen einer Mutation. Auf den nicht-mutierten kann keine PCR durchgeführt werden und demnach gibt es auch zu wenig Edukt für eine LCR, welche in Konsequenz auch nicht zu einem Viskositätsanstieg führt. Eine solche selektive Amplifikation erreicht man mittels allelspezifischer Amplifikation, bei der ein Primer auf die Mutation entwickelt wird. Derartige Verfahren sind in der Fachwelt wohlbekannt.

Denkbar ist auch ein Verfahren, in dem abwechselnd ein PCR-Zyklus und ein LCR-Zyklus oder eine sogenannte Ligationsdetektionsreaktion (LDR) durchlaufen wird.

Als weitere Möglichkeit besteht in der Durchführung einer PCR, gefolgt von einem Oligonukleotid-Ligations-Assay (OLA), bei welcher die Ligation von zwei benachbarten Sonden als einziger Detektionsschritt benützt wird.

All diese Verfahren werden jedoch eine geringere Empfindlichkeit aufweisen als die Kombination von PCR mit der oben genannten speziell adaptierten LCR, die zur weiteren Verkettung d er entstehenden Oligonukleotide führt.

Eine weitere sehr interessante Möglichkeit besteht in der Durchführung einer Gap-LCR anstelle der PCR oder anschliessend an die PCR. In einer Gap-LCR werden komplementäre Sonden-Paare eingesetzt mit einer 3'-Extension. Nachdem diese auf der Zielsequenz-DNA hybridisiert haben, gibt es eine Lücke von einem oder mehreren Basen zur benachbarten Sonde. Eine thermostabile Polymerase (für den Automationsprozess) ohne 3'→5' Exonuklease Aktivität sowie die passenden Nukleotide (schwimmen in Lösung) werden gebraucht, um die Lücke und die resultierenden Sonden mit DNA-Ligase zu verkoppeln. Die Verwendung von Sonden-Duplexen mit nicht-komplementären 3' Extensionen verhindert die Bildung von Zielmolekül-unabhängigen Ligase-Produkten (sogenannte "blunt-end"-Ligation). Es gibt Studien, die zeigen, dass man weniger als 10 Moleküle pro Reaktionsansatz damit detektieren kann.

In einem Alternativversuch wurde anstelle der PCR eine Gap-LCR ausgeführt. Ein Reaktionsgemisch wurde wie folgt angesetzt. Als Reaktionspuffer wurde "Ampligase 10X Reaction Buffer" von Epicentre Technologies verwendet. Dieser enthielt: 200 mM Tris-HCl (pH 8.3), 250 mM KCI, 100 mM MgCl₂, 5 mM NAD, and 0.1% Triton X-100. Zu 50 Mikroliter Reaktionspuffer wurde zugegeben: Je 50 nmol von geeigneten Sonden für die Gap-LCR, 1.5 U DNA-Polymerase ohne 3'→5' Exonuklease Aktivität (aus Thermus flavus) (MBR, Milwaukee, WI), 20 mM K⁺, 1.5 - 5 U Ampligase (Epicentre Technologies). Die Konzentration der Ampligase ist abhängig von der Art der eingesetzten Ampligase. Definition der Einheit U (Units): Eine Einheit U katalysiert die Ligation von 50% der cos-Stellen in 1 Mikrogramm Lambda-DNA in 1 Minute bei 45 °C in "1X Ampligase Reaction Buffer". 1 U Ampligase DNA Ligase ist äquivalent zu mindestens 15 "Cohesive End Units" oder "Nick Ligation Units", wie sie sonst häufig im Fachgebiet verwendet werden. Die Ampligase wurde in einem Aufbewahrungspuffer bereitgestellt, der die folgende Zusammensetzung aufwies: 50% Glycerol mit 50 mM Tris-HCl (pH 7.5), 0.1 M NaCl, 0.1 mM EDTA, 1 mM DTT, und 0.1% Triton X-100. Das Reaktionsvolumen wurde mit 50 Mikroliter Mineralöl überschichtet.

Die weitere Auswertung erfolgte wie im obigen Beispiel. Das Ergebnis war vergleichbar mit demjenigen nach einer PCR.

Denkbar ist auch eine adaptierte Gap-LCR, bei der die Sonden so ausgebildet sind, dass sie mit ihren Enden auf unterschiedlichen Nukleinsäurefragmenten hybridisieren, analog zur oben beschriebenen adaptierten LCR.

Bei den vorgeschlagenen Verfahren resultieren eine Reihe von Vorteilen gegenüber einer konventionellen PCR mit Fluoreszenzdetektion. Darunter sind die folgenden Vorteile besonders hervorzuheben.
- Die Detektion ist sehr kostengünstig (bis zu einem Faktor 20 günstiger als bei optischer Detektion).
- Es ist kein Labeling (Farbstoffkopplung) nötig. Daher wird die Synthese kostengünstiger.
- Es müssen keine speziellen Hybridisationssonden entwickelt werden.
- Die Reaktion kann gepoolt werden, d.h., es können 10, 50, 100 oder noch mehr Patienten simultan getestet werden. Findet keine Viskositätszunahme statt, so weisen alle getesteten Proben keine Mutation auf (falls das Design auf die Mutation erfolgt ist). Registriert man eine Viskositätszunahme, so muss eine oder mehrere Proben mutiert sein. Durch Teilen des Pools kann darauf deduktiv auf die Mutation geschlossen werden.
- Die Reaktion ist automatisierbar: entweder mit einem Pipettierroboter oder mit thermostabiler Ligase.
- Die Reaktion ist vielschichtig. Es gibt einige Subvarianten, die die Anwendung hochinteressant machen wie beispielsweise das Differenzieren von Mikroorganismen. Mikroorganismen mit ähnlichen Sequenzen lassen sich nur schwer mittels allelspezifischer Amplifikation differenzieren. Aber in einem ersten Prozess könnte durchaus eine solche Selektion gemacht werden. In einem folgenden Schritt wird dann das Produkt einer sequenzspezifischen Vernetzung unterworfen. Das könnte z.B. mit einer selektiven Verankerung der Moleküle im Reaktionsgefäss (Well) erfolgen. Die Kopplung und Verankerung geschieht mit Streptavidin / Biotin. Die Capture Probe (komplementär) könnte die entsprechenden Fragmente hybridisieren. Zusätzlich werden jetzt Verzweigungen, welche auf die Sequenzunterschiede der Subspezies entwickelt sind, aktiviert, z.B. mittels Dendrimeren, welche wiederum ein Netzwerk ausbilden und so die Viskosität im Falle eines Vorliegens einer bestimmten Sequenz und damit Species aktiv werden und die Viskosität erhöhen.

Allgemein wird aus dem Vorstehenden klar, dass sich das erfindungsgemässe Verfahren allgemein zur Ermittlung des Reaktionszustands einer Amplifikationsreaktion, Nicht erfindungsgemäß könnte das Verfahren auch für andere viskositätsändernde chemische Reaktionen, insbesondere bei kleinen Probenmengen, verwendet werden.

Auch bei der Vorrichtung sind vielfältige Variationen möglich, ohne den Bereich der Erfindung zu verlassen, wie aus den oben dargestellten Beispielen offensichtlich ist, und die Erfindung ist keineswegs auf diese Beispiele beschränkt.

### Bezugszeichenliste

- 1: Viskosimeter (erste Ausführungsform)
- 101: Glasrohr
- 102, 102': Inertialmasse
- 103, 104: Permanentmagnet
- 105, 106: Elektromagnet
- 107: Gehäuse
- 110: Reaktionsgemisch
- A-A: Schnittebene

- 3: Viskosimeter (zweite Ausführungsform)
- 301: Glasrohr
- 302: Inertialmasse
- 303: Kontaktkörper (permanentmagnetisch)
- 305, 306: Elektromagnet
- 307: Gehäuse
- 310: Reaktionsgemisch
- 311: Öffnung
- B-B: Schnittebene

- 5: Viskosimeter (dritte Ausführungsform)
- 501: Glasrohr
- 502: Inertialmasse
- 503, 503': Kontaktkörper (permanentmagnetisch)
- 505, 506: Elektromagnet
- 507: Gehäuse
- 508, 508': Dosiereinsatz
- 509, 509': Dosierbereich
- 510: Reaktionsgemisch
- 511, 511': Öffnung
- C-C: Schnittebene

- 812,813: Heizelement
- D-D: Schnittebene

- 1012, 1013: Heizelement

- n: Messungsnummer
- η: Viskosität

### Sequenzprotokoll - freier Text

- SEQ ID NO 1: Primer für PCR, "forward"-Strang
- SEQ ID NO 2: Primer für PCR, "reverse"-Strang
- SEQ ID NO 3: Sonde für LCR, Wildtyp-spezifisch, "forward"-Strang
- SEQ ID NO 4: Sonde für LCR, mutationssspezifisch, "forward"-Strang
- SEQ ID NO 5: Sonde für LCR, unspezifisch, 5'-Ende phosphoryliert, "forward"-Strang
- SEQ ID NO 6: Sonde für LCR, Wildtyp-spezifisch, "reverse"-Strang
- SEQ ID NO 7: Sonde für LCR, mutationsspezifisch, "reverse"-Strang
- SEQ ID NO 8: Sonde für LCR, unspezifisch, 5'-Ende phosphoryliert, "reverse"-Strang
- SEQ ID NO 9: Linker-Sonde für adaptierte LCR, Wildtyp-spezifisch, 5'-Ende phosphoryliert, "forward"-Strang
- SEQ ID NO 10: Linker-Sonde für adaptierte LCR, Wildtyp-spezifisch, 5'-Ende phosphoryliert, "reverse"-Strang
- SEQ ID NO 11: Linker-Sonde für adaptierte LCR, mutationsspezifisch, 5'-Ende phosphoryliert, "forward"-Strang
- SEQ ID NO 12: Linker-Sonde für adaptierte LCR, mutationssspezifisch, 5'-Ende phosphoryliert, "reverse"-Strang

## Patentansprüche

1. Verfahren zum Ermitteln des Reaktionszustands eines chemischen Reaktionsvorgangs in einem Reaktionsgemisch (110; 310; 510), wobei der Reaktionsvorgang eine Amplifikationsreaktion für Nukleinsäuren umfasst, **dadurch gekennzeichnet, dass** das Verfahren eine Viskositätsbestimmung des Reaktionsgemisches mit einem Viskosimeter umfasst.

2. Verfahren gemäss Anspruch 1, **dadurch gekennzeichnet, dass** die Amplifikationsreaktion eine Polymerase-Kettenreaktion und/oder Ligase-Kettenreaktion umfasst.

3. Verfahren gemäss Anspruch 1 oder 2, **dadurch gekennzeichnet, dass** die Amplifikationsreaktion eine allelspezifische Amplifikationsreaktion umfasst.

4. Verfahren gemäss einem der Ansprüche 1 bis 3, **dadurch gekennzeichnet, dass** der Reaktionsvorgang eine Verknüpfung von Nukleinsäurefragmenten mittels eines Linker-Oligonukleotids umfasst, das dazu ausgebildet ist, mit einem Bereich nahe seines 5'-Endes auf einem ersten Nukleinsäurefragment zu hybridisieren und mit einem Bereich nahe seinem 3'-Ende auf einem zweiten Nukleinsäurefragment zu hybridisieren.

5. Verfahren gemäss einem der Ansprüche 1 bis 4, **dadurch gekennzeichnet, dass** die Viskositätsbestimmung mit einem dynamischen Viskosimeter (1; 3; 5) erfolgt und die folgenden Schritte umfasst:
- Bereitstellen des dynamischen Viskosimeters (1; 3; 5), das einen Resonator (101, 103, 104; 301, 303; 501, 503) und mindestens einen ersten mit dem Resonator zusammenwirkenden Wandler (103, 104, 105, 106; 303, 305, 306; 503, 505, 506) umfasst, wobei der Resonator (101; 301, 303; 501, 503) zu einer mechanischen Eigenschwingung mit einer Eigenfrequenz fähig ist und eine Kontaktoberfläche aufweist;
- In-Kontakt-Bringen des Reaktionsgemisches (110; 310; 510) mit der Kontaktoberfläche;
- Ermitteln eines Masses für die Viskosität mittels des dynamischen Viskosimeters (1; 3; 5).

6. Verfahren gemäss Anspruch 5, **dadurch gekennzeichnet, dass** das mit der Kontaktoberfläche in Kontakt stehende Reaktionsgemisch (110; 310; 510) sich in einem Probenraum befindet, der ein Volumen aufweist, welches kleiner als 1 Milliliter ist.

7. Verfahren gemäss Anspruch 5 oder 6, **dadurch gekennzeichnet, dass** das Reaktionsgemisch (110; 310; 510) zumindest teilweise durch Kapillarkräfte im Probenraum gehalten wird.

8. Verfahren gemäss einem der vorhergehenden Ansprüche, **dadurch gekennzeichnet, dass** wenigstens ein Teil des Reaktionsvorgangs stattfindet, während sich das Reaktionsgemisch (110; 310; 510) in Kontakt mit der Kontaktoberfläche befindet.

9. Verfahren gemäss Anspruch 5, **dadurch gekennzeichnet, dass** das Viskosimeter ein nahe der Kontaktoberfläche angeordnetes Temperierelement (812, 813; 1012, 1013) umfasst, um das Reaktionsgemisch (110; 310; 510) zu heizen und/oder zu kühlen.

10. Verfahren gemäss Anspruch 9, **dadurch gekennzeichnet, dass** das Temperierelement (812, 813; 1012, 1013) mindestens einen Heizdraht und/oder mindestens ein Peltierelement umfasst und auf dem Resonator (101; 301, 303; 501, 503) angebracht, insbesondere auf diesen aufgedampft, ist.

11. Verfahren gemäss Anspruch 9 oder 10, **dadurch gekennzeichnet, dass** das Temperierelement (812, 813) ein Teil des mindestens einen Wandlers ist.

12. Verfahren gemäss Anspruch 5 oder einem der Ansprüche 9 bis 11, **dadurch gekennzeichnet, dass** der Resonator (101, 103, 104; 301, 303; 501, 503) ein Glasrohr (101; 301; 501) mit einem Aussendurchmesser von 1 Millimeter oder weniger umfasst.

13. Verfahren gemäss Anspruch 12, **dadurch gekennzeichnet, dass** das Viskosimeter eine erste und eine zweite Inertialmasse (102, 102') aufweist, an denen das Glasrohr (101) vorzugsweise lösbar befestigt ist, dass der Wandler (103, 104, 105, 106) im Bereich zwischen der ersten und der zweiten Inertialmasse (102, 102') nahe einem Schwingungsantiknoten der Eigenschwingung des Resonators (101, 103, 104) angeordnet ist, und dass das Innere des Glasrohrs (101) die Kontaktoberfläche bildet.

14. Verfahren gemäss Anspruch 5 oder einem der Ansprüche 9 bis 13, **dadurch gekennzeichnet, dass** der Resonator (301, 303; 501, 503) ein Rohr (301; 501) und einen Kontaktkörper (303; 503) aufweist, der mit dem Rohr (301; 501) verbunden ist und einen Aussendurchmesser aufweist, der grösser ist als ein Aussendurchmesser des Rohrs (301; 501), dass die Kontaktoberfläche wenigstens einen Teil der äusseren Oberfläche des Kontaktkörpers (303; 503) umfasst, und dass der Kontaktkörper (303; 503) zu einer elektromagnetischen Wechselwirkung mit einem vom Wandler (303, 305, 306; 503, 505, 506) erzeugten Magnetfeld ausgebildet ist.

15. Verfahren gemäss Anspruch 5 oder einem der Ansprüche 9 bis 14, **dadurch gekennzeichnet, dass** das Viskosimeter eine Dosiervorrichtung (307; 508) aufweist, die nahe der Kontaktoberfläche angeordnet ist und die im Bereich der Kontaktoberfläche eine Öffnung (311; 511) aufweist, um das Reaktionsgemisch der Kontaktoberfläche zuzuführen, und dass die Dosiervorrichtung (307; 508) so von der Kontaktoberfläche beabstandet ist, dass ein in einen vorbestimmten Bereich zwischen der Kontaktoberfläche und der Dosiervorrichtung eingebrachtes Reaktionsgemisch durch Kapillarkräfte in dem vorbestimmten Bereich gehalten wird.

16. Verfahren gemäss Anspruch 15, **dadurch gekennzeichnet, dass** der Resonator (301, 303; 501, 503) ein Rohr (301; 501) und einen Kontaktkörper (303; 503) aufweist, der mit dem Rohr (301; 501) verbunden ist und einen Aussendurchmesser aufweist, der grösser ist als ein Aussendurchmesser des Rohrs (301; 501), dass die Kontaktoberfläche wenigstens einen Teil der äusseren Oberfläche des Kontaktkörpers (303; 503) umfasst, und dass die Öffnung im wesentlichen konzentrisch mit dem Rohr (301; 501) angeordnet ist.

17. Verfahren gemäss Anspruch 5 oder einem der Ansprüche 9 bis 16, **dadurch gekennzeichnet, dass** das Viskosimeter eine Rückkopplungseinrichtung aufweist, die einen den ersten Wandler treibenden Oszillator umfasst, wobei die Rückkopplungseinrichtung dazu vorgesehen ist, den Resonator im Bereich einer Eigenfrequenz zu stabilisieren, wobei ein Ausgang der Rückkopplungseinrichtung zumindest indirekt mit dem ersten Wandler verbunden ist und ein Eingang der Rückkopplungsvorrichtung zumindest indirekt mit dem ersten Wandler oder einem zweiten mit dem Resonator verbundenen Wandler verbunden ist.

18. Verfahren gemäss Anspruch 17, **dadurch gekennzeichnet, dass** zwischen dem Ausgang der Rückkopplungsvorrichtung und dem ersten Wandler ein erster Schalter und zwischen dem ersten bzw. zweiten Wandler und dem Eingang der Rückkopplungsvorrichtung ein zweiter Schalter vorhanden sind.

19. Verfahren gemäss Anspruch 1 zur Analyse einer chemischen Reaktion, die eine Polymerase-Kettenreaktion umfasst und zur Analyse von DNA dient, **dadurch gekennzeichnet, dass** das Resultat oder der Stand der Reaktion durch eine Viskositätsmessung ermittelt wird.

20. Verfahren gemäss Anspruch 19, **dadurch gekennzeichnet, dass** die Viskositätsmessung mit einem dynamischen Viskosimeter erfolgt.

21. Verfahren gemäss Anspruch 19 oder 20, **dadurch gekennzeichnet, dass** spezifische biochemische Reagenzien dem Ausgangsprodukt beigemischt werden, um die Viskositätsänderung, die sich mit zunehmendem Fortschreiten der Reaktion einstellt, zu vergrössern.

22. Verfahren gemäss Anspruch 20, **dadurch gekennzeichnet, dass** das dynamische Viskosimeter getaktet betrieben wird.

23. Verfahren gemäss einem der Ansprüche 20 bis 22, **dadurch gekennzeichnet, dass** das dynamische Viskosimeter ein Rohr umfasst.

24. Verfahren gemäss Anspruch 23, **dadurch gekennzeichnet, dass** das Rohr sowohl für die thermischen Zyklen als auch für die Viskositätsmessung verwendet wird.

## Claims

1. A method for determining the reaction status of a chemical reaction process in a reaction mixture (110; 310; 510), wherein the reaction process comprises a reaction for amplifying nucleic acids, **characterized in that** the method comprises a determination of the viscosity of the reaction mixture using a viscometer.

2. The method as claimed in claim 1, **characterized in that** the amplification reaction comprises a polymerase chain reaction and/or ligase chain reaction.

3. The method as claimed in claim 1 or 2, **characterized in that** the amplification reaction comprises an allele-specific amplification reaction.

4. The method as claimed in any of claims 1 to 3, **characterized in that** the reaction process comprises a linkage of nucleic acid fragments by means of a linker oligonucleotide which is designed to hybridize with one region near its 5' end on a first nucleic acid fragment, and to hybridize with a region near its 3' end on a second nucleic acid fragment.

5. The method as claimed in any of claims 1 to 4, **characterized in that** the determination of the viscosity is carried out using a dynamic viscometer (1; 3; 5) and comprises the following steps:
- provision of the dynamic viscometer (1; 3; 5) which comprises a resonator (101, 103, 104; 301, 303; 501, 503) and at least a first transducer (103, 104, 105, 106; 303, 305, 306; 503, 505, 506) cooperating with the resonator, where the resonator (101; 301, 303; 501, 503) is capable of a mechanical natural vibration with a characteristic frequency, and has a contact surface;
- contacting the reaction mixture (110; 310; 510) with the contact surface;
- determining a measure of the viscosity by means of the dynamic viscometer (1; 3; 5).

6. The method as claimed in claim 5, **characterized in that** the reaction mixture (110; 310; 510) in contact with the contact surface is located in a sample chamber which has a volume which is less than 1 milliliter.

7. The method as claimed in claim 5 or 6, **characterized in that** the reaction mixture (110; 310; 510) is at least partly held by capillary forces in the sample chamber.

8. The method as claimed in any of the preceding claims, **characterized in that** at least part of the reaction process takes place while the reaction mixture (110; 310; 510) is in contact with the contact surface.

9. The method as claimed in claim 5, **characterized in that** the viscometer comprises a temperature-control element (812, 813; 1012, 1013) disposed near the contact surface in order to heat and/or cool the reaction mixture (110; 310; 510).

10. The method as claimed in claim 9, **characterized in that** the temperature-control element (812, 813; 1012, 1013) comprises at least one heating wire and/or at least one Peltier element and is attached to the resonator (101; 301, 303; 501, 503), in particular is vapor-deposited thereon.

11. The method as claimed in claim 9 or 10, **characterized in that** the temperature-control element (812, 813) is part of the at least one transducer.

12. The method as claimed in claim 5 or in any of claims 9 to 11, **characterized in that** the resonator (101, 103, 104; 301, 303; 501, 503) comprises a glass tube (101; 301; 501) with an external diameter of 1 millimeter or less.

13. The method as claimed in claim 12, **characterized in that** the viscometer has a first and a second inertial mass (102, 102') to which the glass tube (101) is fastened, preferably detachably, **in that** the transducer (103, 104, 105, 106) is disposed in the region between the first and the second inertial mass (102, 102') close to a vibration antinode of the natural vibration of the resonator (101, 103, 104), and **in that** the interior of the glass tube (101) forms the contact surface.

14. The method as claimed in claim 5 or in any of claims 9 to 13, **characterized in that** the resonator (301, 303; 501, 503) has a tube (301; 501) and a contact body (303; 503) which is connected to the tube (301; 501) and has an external diameter which is greater than an external diameter of the tube (301; 501), **in that** the contact surface comprises at least part of the external surface of the contact body (303; 503), and **in that** the contact body (303; 503) is designed for an electromagnetic interaction with a magnetic field generated by the transducer (303, 305, 306; 503, 505, 506).

15. The method as claimed in claim 5 or in any of claims 9 to 14, **characterized in that** the viscometer has a dosing device (307; 508) which is disposed near the contact surface and which has in the region of the contact surface an orifice (311; 511) in order to feed the reaction mixture to the contact surface, and **in that** the dosing device (307; 508) is at a distance from the contact surface such that a reaction mixture introduced into a predetermined region between the contact surface and the dosing device is held in the predetermined region by capillary forces.

16. The method as claimed in claim 15, **characterized in that** the resonator (301, 303; 501, 503) comprises a tube (301; 501) and a contact body (303; 503) being connected to the tube (301; 501) and having an external diameter which is greater than an external diameter of the tube (301; 501), **in that** the contact surface comprises at least part of the external surface of the contact body (303; 503), and **in that** the orifice is disposed substantially concentrically with the tube (301; 501).

17. The method as claimed in claim 5 or in any of claims 9 to 16, **characterized in that** the viscometer has a feedback unit which comprises an oscillator driving the first transducer, where the feedback unit is intended to stabilize the resonator in the region of a characteristic frequency, where an output terminal of the feedback unit is connected at least indirectly to the first transducer, and an input terminal of the feedback device is connected at least indirectly to the first transducer or to a second transducer connected to the resonator.

18. The method as claimed in claim 17, **characterized in that** a first switch is present between the output terminal of the feedback device and the first transducer, and a second switch is present between the first or second transducer and the input terminal of the feedback device.

19. The method as claimed in claim 1 for analyzing a chemical reaction which includes a polymerase chain reaction and serves for analysis of DNA, **characterized in that** the result or status of the reaction is determined by a viscosity measurement.

20. The method as claimed in claim 19, **characterized in that** the viscosity measurement is carried out with a dynamic viscometer.

21. The method as claimed in claim 19 or 20, **characterized in that** specific biochemical reagents are mixed with the starting material in order to increase the alteration in viscosity which occurs with the advancement the reaction process.

22. The method as claimed in claim 20, **characterized by** gated operation of the dynamic viscometer.

23. The method as claimed in any of claims 20 to 22, **characterized in that** the dynamic viscometer comprises a tube.

24. The method as claimed in claim 23, **characterized in that** the tube is used both for the thermal cycles and for measuring the viscosity.

## Revendications

1. Procédé de détermination de l'état de réaction d'un processus réactionnel chimique dans un mélange réactionnel (110 ; 310 ; 510), le processus réactionnel comprenant une réaction d'amplification d'acides nucléiques, **caractérisé en ce que** le procédé comprend une détermination de la viscosité du mélange réactionnel à l'aide d'un viscosimètre.

2. Procédé selon la revendication 1, **caractérisé en ce que** la réaction d'amplification comprend une réaction en chaîne par polymérase et/ou une réaction en chaîne par ligase.

3. Procédé selon la revendication 1 ou 2, **caractérisé en ce que** la réaction d'amplification comprend une réaction d'amplification spécifique d'allèle.

4. Procédé selon l'une des revendications 1 à 3, **caractérisé en ce que** le processus réactionnel est une liaison de fragments d'acides nucléiques à l'aide d'un oligonucléotide lieur, qui est configuré de façon à s'hybrider, par une zone proche de son extrémité 5', à un premier fragment d'acide nucléique, et, par une zone proche de son extrémité 3', à s'hybrider à un deuxième fragment d'acide nucléique.

5. Procédé selon l'une des revendications 1 à 4, **caractérisé en ce que** la détermination de la viscosité s'effectue à l'aide d'un viscosimètre dynamique (1; 3; 5) et comprend les étapes suivantes:
- mise à disposition du viscosimètre dynamique (1 ; 3 ; 5), qui comprend un résonateur (101, 103, 104 ; 301, 303 ; 501, 503) et au moins un premier transducteur (103, 104, 105, 106 ; 303, 305, 306 ; 503, 505, 506) coopérant avec le résonateur, le résonateur (101 ; 301, 303 ; 501, 503) pouvant avoir une oscillation mécanique propre ayant une fréquence propre, et présentant une surface de contact ;
- mise en contact du mélange réactionnel (110 ; 310 ; 510) avec la surface de contact ;
- détermination d'une mesure de la viscosité à l'aide du viscosimètre dynamique (1 ; 3 ; 5).

6. Procédé selon la revendication 5, **caractérisé en ce que** le mélange réactionnel (110 ; 310 ; 510) en contact avec la surface de contact se trouve dans une chambre à échantillon dont le volume est inférieur à 1 millilitre.

7. Procédé selon la revendication 5 ou 6, **caractérisé en ce que** le mélange réactionnel (110 ; 310 ; 510) est maintenu dans la chambre à échantillon au moins en partie par des forces capillaires.

8. Procédé selon l'une des revendications précédentes, **caractérisé en ce qu'**au moins une partie du processus réactionnel a lieu pendant que le mélange réactionnel (110 ; 310 ; 510) se trouve en contact avec la surface de contact.

9. Procédé selon la revendication 5, **caractérisé en ce que** le viscosimètre possède un élément (812, 813 ; 1012, 1013) de mise à l'équilibre de température, disposé au voisinage de la surface de contact, pour chauffer et/ou refroidir le mélange réactionnel (110 ; 310 ; 510).

10. Procédé selon la revendication 9, **caractérisé en ce que** l'élément de mise à l'équilibre de température (812, 813 ; 1012, 1013) comprend au moins un fil chauffant et/ou au moins un élément Peltier, et est appliqué sur le résonateur (101 ; 301, 303 ; 501, 503), en particulier par dépôt en phase vapeur.

11. Procédé selon la revendication 9 ou 10, **caractérisé en ce que** l'élément de mise à l'équilibre de température (812, 813) est une partie de l'au moins un transducteur.

12. Procédé selon la revendication 5 ou l'une des revendications 9 à 11, **caractérisé en ce que** le résonateur (101, 103, 104 ; 301, 303 ; 501, 503) comprend un tube de verre (101 ; 301 ; 501) ayant un diamètre extérieur de 1 millimètre ou moins.

13. Procédé selon la revendication 12, **caractérisé en ce que** le viscosimètre comprend une première et une deuxième masses inertielles (102, 102'), auxquelles le tube de verre (101) peut être fixé, de préférence d'une manière amovible, que le transducteur (103, 104, 105, 106) est disposé dans la zone située entre la première et la deuxième masses inertielles (102, 102'), au voisinage d'un ventre de l'oscillation propre du résonateur (101, 103, 104), et que l'intérieur du tube de verre (101) forme la surface de contact.

14. Procédé selon la revendication 5 ou l'une des revendications 9 à 13, **caractérisé en ce que** le résonateur (301, 303 ; 501, 503) comprend un tube (301 ; 501) et un corps de contact (303 ; 503), qui est relié au tube (301 ; 501) et présente un diamètre extérieur qui est supérieur à un diamètre extérieur du tube (301 ; 501), que la surface de contact comprend au moins une partie de la surface extérieure du corps de contact (303 ; 503), et que le corps de contact (303 ; 503) est configuré pour réaliser une interaction électromagnétique avec un champ magnétique produit par le transducteur (303, 305, 306 ; 503, 505, 506).

15. Procédé selon la revendication 5 ou l'une des revendications 9 à 14, **caractérisé en ce que** le viscosimètre comprend un dispositif doseur (307 ; 508), qui est disposé au voisinage de la surface de contact et présente, dans la zone de la surface de contact, une ouverture (311 ; 511) pour amener le mélange réactionnel à la surface de contact, et que le dispositif doseur (307 ; 508) se trouve à une distance de la surface de contact telle qu'un mélange réactionnel introduit dans une zone prédéfinie entre la surface de contact et le dispositif doseur est maintenu dans la zone prédéfinie sous l'effet de forces capillaires.

16. Procédé selon la revendication 15, **caractérisé en ce que** le résonateur (301, 303 ; 501, 503) comprend un tube (301 ; 501) et un corps de contact (303 ; 503), qui est relié au tube (301 ; 501) et présente un diamètre extérieur qui est supérieur à un diamètre extérieur du tube (301 ; 501), que la surface de contact comprend au moins une partie de la surface extérieure du corps de contact (303 ; 503), et que l'ouverture est disposée d'une manière essentiellement concentrique au tube (301 ; 501).

17. Procédé selon la revendication 5 ou l'une des revendications 9 à 16, **caractérisé en ce que** le viscosimètre comprend un dispositif d'auto-excitation, qui comprend un oscillateur agissant sur le premier transducteur, le dispositif d'auto-excitation étant prévu de façon à stabiliser le résonateur dans la zone d'une fréquence propre, une sortie du dispositif d'auto-excitation étant au moins indirectement reliée au premier transducteur, et une entrée du dispositif d'auto-excitation étant au moins indirectement reliée au premier transducteur ou à un deuxième transducteur relié au résonateur.

18. Procédé selon la revendication 17, **caractérisé en ce qu'**un premier commutateur est présent entre la sortie du dispositif d'auto-excitation et le premier transducteur, et qu'un deuxième commutateur est présent entre le premier ou le deuxième transducteur et l'entrée du dispositif d'auto-excitation.

19. Procédé selon la revendication 1, pour l'analyse d'une réaction chimique comprenant une réaction en chaîne par polymérase, et qui sert à l'analyse de l'ADN, **caractérisé en ce que** le résultat ou l'état de la réaction est déterminé par une mesure de la viscosité.

20. Procédé selon la revendication 19, **caractérisé en ce que** la mesure de la viscosité s'effectue à l'aide d'un viscosimètre dynamique.

21. Procédé selon la revendication 19 ou 20, **caractérisé en ce que** des réactifs biochimiques spécifiques sont mélangés au produit de départ, pour augmenter la variation de viscosité qui se met en place au fur et à mesure de l'avancement de la réaction.

22. Procédé selon la revendication 20, **caractérisé en ce que** le viscosimètre dynamique est exploité par impulsions d'horloge.

23. Procédé selon l'une des revendications 20 à 22, **caractérisé en ce que** le viscosimètre dynamique comprend un tube.

24. Procédé selon la revendication 23, **caractérisé en ce que** le tube est utilisé tant pour les cycles thermiques que pour la mesure de la viscosité.


### Sequenzprotokoll

<110> Dual, Jürg
   Bestmann, Lukas
   Häusler, Klaus
<120> Chemische Analyse mit dynamischer Viskosimetrie
<130> P142203
<150> CH 1923/03<151> 2003-11-06
<160> 12
<170> Patentin version 3.1
<210> 1
   <211> 20
   <212> DNA
   <213> Artificial Sequence
<220>
   <223> Primer for PCR, forward strand
<400> 1
   taatctgtaa gagcagatcc 20
<210> 2
   <211> 20
   <212> DNA
   <213> Artificial Sequence
<220>
   <223> Primer for PCR, reverse strand
<400> 2
   tgttatcaca ctggtgctaa 20
<210> 3
   <211> 17
   <212> DNA
   <213> Artificial Sequence
<220>
   <223> Probe for LCR, wildtype-specific, forward strand
<400> 3
   agatccctgg acaggcg 17
<210> 4
   <211> 18
   <212> DNA
   <213> Artificial Sequence
<220>
   <223> Probe for LCR, mutation-specific, forward strand
<400> 4
   cagatccctg gacaggca 18
<210> 5
   <211> 25
   <212> DNA
   <213> Artificial Sequence
<220>
   <223> Probe for LCR, unspecific, 5' end phosphorylated, forward strand
<400> 5
   aggaatacag gtattttgtc cttga 25
<210> 6
   <211> 25
   <212> DNA
   <213> Artificial Sequence
<220>
   <223> Probe for LCR, wildtype-specific, reverse strand
<400> 6
   caaggacaaa atacctgtat tcctc 25
<210> 7
   <211> 25
   <212> DNA
   <213> Artificial Sequence
<220>
   <223> Probe for LCR, mutation-specific, reverse strand
<400> 7
   caaggacaaa atacctgtat tcctt 25
<210> 8
   <211> 17
   <212> DNA
   <213> Artificial Sequence
<220>
   <223> Probe for LCR, unspecific, 5' end phosphorylated, forward strand
<400> 8
   gcctgtccag ggatctg 17
<210> 9
   <211> 51
   <212> DNA
   <213> Artificial Sequence
<220>
   <223> Linker probe for adapted LCR, wildtype-specific, 5' end phosphorylated, forward strand
<400> 9
   aggaatacag gtattttgtc cttgattttt ttttagatcc ctggacaggc g 51
<210> 10
   <211> 51
   <212> DNA
   <213> Artificial Sequence
<220>
   <223> Linker probe for adapted LCR, wildtype-specific, 5' end phosphorylated, reverse strand
<400> 10
   gcctgtccag ggatctgttt ttttttcaag gacaaaatac ctgtattcct c 51
<210> 11
   <211> 51
   <212> DNA
   <213> Artificial Sequence
<220>
   <223> Linker probe for adapted LCR, mutation-specific, 5' end phosphorylated, forward strand
<400> 11
   aggaatacag gtattttgtc cttgattttt ttttagatcc ctggacaggc a 51
<210> 12
   <211> 51
   <212> DNA
   <213> Artificial Sequence
<220>
   <223> Linker probe for adapted LCR, mutation-specific, 5' end phosphorylated, reverse strand
<400> 12
   gcctgtccag ggatctgttt ttttttcaag gacaaaatac ctgtattcct t 51